(19) **Europäisches Patentamt European Patent Office Office européen des brevets**

(11) **EP 3 773 242 B1**

(12) **EUROPEAN PATENT SPECIFICATION**

(45) Date of publication and mention of the grant of the patent:
**03.09.2025 Bulletin 2025/36**

(21) Application number: **19774321.4**

(22) Date of filing: **27.03.2019**

(51) International Patent Classification (IPC):
**A61B 17/00** *(2006.01)*  **A61B 17/32** *(2006.01)*
**A61B 34/00** *(2016.01)*  **A61B 34/30** *(2016.01)*
**A61B 9/00** *(2006.01)*  **A61B 17/29** *(2006.01)*
**A61B 17/3201** *(2006.01)*  **A61B 18/14** *(2006.01)*

(52) Cooperative Patent Classification (CPC):
**A61B 17/0467; A61B 17/0469; A61B 17/062;**
**A61B 17/29; A61B 17/3201; A61B 18/1445;**
**A61B 34/37; A61B 34/71; A61G 13/04;**
**A61G 13/06; A61G 13/10;** A61B 2017/00017;
A61B 2017/00353; A61B 2017/00398;
A61B 2017/00477;            (Cont.)

(86) International application number:
**PCT/US2019/024333**

(87) International publication number:
**WO 2019/191265 (03.10.2019 Gazette 2019/40)**

(54) **ROBOTICALLY-ENABLED MEDICAL SYSTEMS WITH MULTIFUNCTION END EFFECTORS HAVING ROTATIONAL OFFSETS**

ROBOTERFÄHIGE MEDIZINISCHE SYSTEME MIT MULTIFUNKTIONALEN ENDEFFEKTOREN MIT ROTATIONS-OFFSETS

SYSTÈMES MÉDICAUX ACTIVÉS PAR ROBOT COMPRENANT DES EFFECTEURS TERMINAUX MULTIFONCTIONS AYANT DES DÉCALAGES EN ROTATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.03.2018 US 201862650190 P**

(43) Date of publication of application:
**17.02.2021 Bulletin 2021/07**

(73) Proprietor: **Auris Health, Inc.**
**Redwood City CA 94065 (US)**

(72) Inventors:
 • **CHIN, Erica Ding**
  **Redwood City, California 94065 (US)**
 • **SCHUH, Travis Michael**
  **Redwood City, California 94065 (US)**
 • **MARSOT, Travis R.**
  **Redwood City, California 94065 (US)**
 • **DOISNEAU, Anne Donahue**
  **Redwood City, California 94065 (US)**

(74) Representative: **Carpmaels & Ransford LLP**
**One Southampton Row**
**London WC1B 5HA (GB)**

(56) References cited:
| | |
|---|---|
| EP-A1- 3 466 347 | WO-A1-2019/069180 |
| US-A- 5 545 170 | US-A1- 2007 244 515 |
| US-A1- 2013 053 877 | US-A1- 2014 005 681 |
| US-A1- 2015 313 676 | US-A1- 2017 095 295 |
| US-B2- 8 820 603 | US-B2- 8 882 660 |

(52) Cooperative Patent Classification (CPC): (Cont.)
A61B 2017/00809; A61B 2017/2926;
A61B 2017/2946; A61B 2017/2947;
A61B 2018/1422; A61B 2018/1457; A61B 2018/146;
A61B 2034/105; A61B 2034/2051; A61B 2034/2055;
A61B 2034/2059; A61B 2034/2061;
A61B 2034/2065; A61B 2034/301; A61B 2034/302;
A61B 2034/305; A61B 2090/0801; A61B 2090/306;
A61B 2090/309; A61B 2090/3614

## Description

### TECHNICAL FIELD

This application relates to robotically-enabled medical systems, and in particular, to robotically-enabled medical systems with multifunction end effectors having rotational offsets.

### BACKGROUND

Medical procedures such as laparoscopy may involve accessing and visualizing an internal region of a patient. In a laparoscopic procedure, a medical instrument can be inserted into the internal region through a laparoscopic access port. The medical instrument can include an end effector configured to perform a function during the procedure.

In certain procedures, a robotically-enabled medical system may be used to control the insertion and/or manipulation of the instrument and end effector. The robotically-enabled medical system may include a robotic arm, or other instrument positioning device, having a manipulator assembly used to control the positioning of the instrument and end effector during the procedure.

US2015/313676A1 describes a wristed surgical instrument capable of multiple functions without requiring extra inputs. US2013/053877A1 describes a multiple function surgical instrument. US5545170 describes a surgical instrument. US2007/244515A1 describes a multipurpose surgical tool.

### SUMMARY

The invention is defined by the appended independent claim 1. Further embodiments of the invention are defined in the dependent claims. Medical systems, such as robotically-enabled medical systems, with multifunction end effectors having rotational offsets are described herein. In a first aspect, a medical system includes a medical instrument including an end effector. The end effector includes a first body that shares a pivot axis with a second body. The first body is rotatable relative to the second body to permit a first mode of operation and a second mode of operation. In some embodiments, during the first mode of operation the second mode of operation is inoperable. In some embodiments, the first mode of operation and second mode of operation can be controlled substantially about one axis.

The system may include one or more of the following features in any combination: (a) wherein the first mode of operation comprises a gripping mode and the second mode of operation comprises a cutting mode; (b) wherein the first body includes a first engagement surface having a first portion rotationally offset from a second portion, and wherein the second body includes a second engagement surface having a first portion rotationally offset from a second portion; (c) at least one non-transitory computer readable medium having stored thereon executable instruments, and at least one processor in communication with the at least one non-transitory computer readable medium and configured to execute the instructions to cause the system to at least: operate the end effector in the first mode of operation to permit cooperation between the first portion of the first engagement surface and the first portion of the second engagement surface and preclude cooperation between the second portion of the first engagement surface and the second portion of the second engagement surface, transition the end effector from the first mode of operation to the second mode of operation to permit cooperation between at least the second portion of the first engagement surface and the second portion of the second engagement surface, and operate the end effector in the second mode of operation; (d) wherein the first portion of the first body is rotationally offset from the second portion of the first body by at least 10 degrees; (e) wherein the first body includes a first portion and a second portion, wherein the first portion comprises a gripping portion and the second portion comprises a cutting portion; (f) wherein during the first mode of operation, the cutting portion is unexposed; (g) wherein both the first body and the second body comprise a gripping portion and a cutting portion, and wherein during the first mode of operation, the cutting portions overlap; (h) wherein during the second mode of operation, a recess is formed between the cutting portion; (i) wherein in the first mode of operation, the end effector comprises a mechanical stop that makes the second mode of operation inoperable; (j) wherein the mechanical stop comprises a hard stop formed on the first body and the second body of the end effector; and/or (k) a robotic arm coupled to the medical instrument.

In another aspect, a medical system includes a medical instrument including an end effector. The end effector includes a first body and a second body, wherein the end effector comprises a first mode of operation and a second mode of operation, wherein during the first mode of operation the second mode of operation is hidden, and wherein the first mode of operation and second mode of operation can be controlled substantially about one axis.

In some embodiments, the system includes one or more of the following features in any combination: (a) wherein the first mode of operation comprises active gripping and the second mode of operation comprises active cutting; (b) a robotic arm coupled to the medical instrument; and at least one processor configured to execute instructions to cause the medical system to at least operate the end effector in the first mode of operation, and preclude operation of the end effector in the second mode of operation; and/or (c) wherein the processor is configured to transition the end effector from the first mode of operation to the second mode of operation and operate the end effector in the second mode of operation.

In another aspect, a medical system is described. The system includes a robotically controlled end effector for a robotic surgical instrument. The end effector includes a first body including a first engagement surface having a first portion rotationally offset from a second portion, a second body including a second engagement surface having a first portion rotationally offset from a second portion, and a pivot axis substantially shared by the first body and the second body, the first body and the second body configured for the rotational movement about the pivot axis. The system also includes at least one non-transitory computer readable medium having stored thereon executable instructions, and at least one processor in communication with the at least one non-transitory computer readable medium. The at least one processor is configured to execute the instructions to cause the system to at least: operate the end effector in a first mode of operation to permit cooperation between the first portion of the first engagement surface and the first portion of the second engagement surface and preclude cooperation between the second portion of the first engagement surface and the second portion of the second engagement surface, transition the end effector from the first mode of operation to a second mode of operation to permit cooperation between at least the second portion of the first engagement surface and the second portion of the second engagement surface, and operate the end effector in the second mode of operation.

In some embodiments, the system includes one or more of the following features in any combination: (a) wherein the first mode of operation comprises a gripping mode and the second mode of operation comprises a cutting model; (b) wherein when the end effector is in the gripping mode, the cutting mode is inoperable; (c) wherein the first portion of the first body comprises a gripping portion and the second portion of the first body comprises a cutting portion; (d) wherein during the first mode of operation, the cutting portion is unexposed; (e) wherein both the first body and the second body comprise a gripping portion and a cutting portion, and wherein during the first mode of operation, the cutting portions overlap.; (f) wherein during the second mode of operation, a recess is formed between the cutting portions; (g) wherein the first portion is rotationally offset from the second portion by at least 15 degrees; (h) wherein the first portion is rotationally offset from the second portion by at least 10 degrees; (i) wherein the first mode of operation and the second mode of operation can be robotically controlled; (j) wherein the instructions further configure the processor to determine whether the end effector is in the first mode of operation or the second mode of operation; (k) wherein, in the first mode of operation, the end effector comprises a mechanical stop that makes the second mode of operation inoperable; (l) wherein the mechanical stop comprises a hard stop formed on the bodies of the end effector; (m) wherein in the first mode of operation, a master comprises a mechanical stop that makes the second mode of operation inoperable; (n) wherein each of the first body and the second body comprise a gripping portion and a cutting portion, and wherein a distance of separation between the gripping portions of the first body and the second body is less in the first mode than in the second mode; (o) wherein each of the first body and the second body comprise a gripping portion and a cutting portion, and wherein an angle of separation between the gripping portions of the first body and the second body is less in the first mode than in the second mode; (p) wherein each of the first body and the second body comprise a gripping portion and a cutting portion, and wherein in the first mode of operation, edges of the cutting portions remain fully overlapped; and/or (q) wherein the instructions further configure the processor to: transition the end effector from the first mode of operation to a second mode of operation to permit cooperation between at least the second portion of the first engagement surface and the second portion of the second engagement surface, and operate the end effector in the second mode of operation.

In another aspect, an end effector for a robotic surgical instrument includes a first mode of operation, and a second mode of operation, wherein during the first mode of operation, the second mode of operation is inoperable, and wherein the first mode of operation and second mode of operation can be controlled substantially about one axis.

In another aspect, an end effector for a robotic surgical instrument includes a first body that shares a pivot axis with the second body, the first body rotatable relative to the second body to permit a first mode of operation and a second mode of operation, wherein during the first mode of operation the second mode of operation is inoperable, and wherein the first mode of operation and second mode of operation can be controlled substantially about one axis.

In another aspect, an end effector for a robotic surgical instrument, the end effector includes a first body including a first engagement surface having a first portion rotationally offset from a second portion, a second body including a second engagement surface having a first portion rotationally offset from a second portion, and a pivot operatively coupling the first body to the second body, the pivot permitting rotational movement of the first body and the second body about the pivot such that the end effector is operable in at least: a first mode of operation permitting cooperation between the first portion of the first engagement surface with the first portion of the second engagement surface, and precluding cooperation between the second portion of the first engagement surface and the second portion of the second engagement surface, and a second mode of operation permitting cooperation between at least the second portion of the first engagement surface and the second portion of the second engagement surface.

In another aspect, an end effector for a robotic surgical instrument includes a first body and a second body, wherein the end effector comprises a first mode of operation and a second mode of operation, wherein during the first mode of operation the second mode of operation is hidden, and wherein the first mode of operation and second mode of operation can be controlled substantially about one axis.

In another aspect, an end effector for a robotic surgical instrument includes a first body rotationally related to a second body by a shared pivot axis, the pivot axis permitting rotation between the first body and the second body to allow operation in at least a first mode of operation and a second mode of operation, wherein the second mode of operation is hidden during the first mode of operation, and wherein the first mode of operation and second mode of operation can be controlled substantially about one axis.

In another aspect, an end effector for a robotic surgical instrument includes a first body including a first engagement surface having a first portion rotationally offset from a second portion, a second body including a second engagement surface having a first portion rotationally offset from a second portion, and a pivot operably coupling the first body to the second body, the pivot permitting rotational movement of the first body and the second body about the pivot such that the end effector is operable in at least: a first rotational range in which the second portion of the first engagement surface overlaps the second portion of the second engagement surface precluding access to the second portion of the first engagement surface and the second portion of the second engagement surface and permitting access to the first portion of the first engagement surface and the first portion of the second engagement surface, and a second rotational range permitting access to at least the first portion of the first engagement surface and the second portion of the second engagement surface.

In another aspect, a non-transitory computer readable storage medium is described. The non-transitory computer readable medium includes stored thereon instructions that, when executed, cause a processor of a device to at least: operate a surgical end effector in a first mode of operation; and operate the surgical end effector in a second mode of operation, wherein during the first mode of operation the second mode of operation is inoperable, and wherein the first mode of operation and second mode of operation can be controlled about one axis.

In some embodiments, the non-transitory computer readable medium includes one or more of the following features in any combination: (a) wherein the first mode of operation comprises active gripping and the second mode comprises active cutting; (b) wherein during the first mode of operation, active cutting is unavailable; (c) wherein during the second mode of operation, active cutting is available; (d) wherein during the second mode of operation, active gripping and active cutting are available; and/or (e) wherein modifying the end effector from the first mode of operation to a second mode of operation is performed via software.

## BRIEF DESCRIPTION OF THE DRAWINGS

The disclosed aspects will hereinafter be described in conjunction with the appended drawings, provided to illustrate and not to limit the disclosed aspects, wherein like designations denote like elements.

FIG. 1 illustrates an embodiment of a cart-based robotic system arranged for diagnostic and/or therapeutic bronchoscopy procedure(s).

FIG. 2 depicts further aspects of the robotic system of FIG. 1.

FIG. 3 illustrates an embodiment of the robotic system of FIG. 1 arranged for ureteroscopy.

FIG. 4 illustrates an embodiment of the robotic system of FIG. 1 arranged for a vascular procedure.

FIG. 5 illustrates an embodiment of a table-based robotic system arranged for a bronchoscopy procedure.

FIG. 6 provides an alternative view of the robotic system of FIG. 5.

FIG. 7 illustrates an example system configured to stow robotic arm(s).

FIG. 8 illustrates an embodiment of a table-based robotic system configured for a ureteroscopy procedure.

FIG. 9 illustrates an embodiment of a table-based robotic system configured for a laparoscopic procedure.

FIG. 10 illustrates an embodiment of the table-based robotic system of FIGs. 5-9 with pitch or tilt adjustment.

FIG. 11 provides a detailed illustration of the interface between the table and the column of the table-based robotic system of FIGs. 5-10.

FIG. 12 illustrates an exemplary instrument driver.

FIG. 13 illustrates an exemplary medical instrument with a paired instrument driver.

FIG. 14 illustrates an alternative design for an instrument driver and instrument where the axes of the drive units are parallel to the axis of the elongated shaft of the instrument.

FIG. 15 depicts a block diagram illustrating a localization system that estimates a location of one or more elements of the robotic systems of FIGs. 1-10, such as the location of the instrument of FIGs. 13 and 14, in accordance to an example embodiment.

FIG. 16A illustrates an embodiment of a multifunction end effector performing a suturing procedure.

FIG. 16B illustrates inadvertent contact between a suture thread and a cutting portion of the multifunction end effector of FIG. 16B.

FIG. 17 illustrates an embodiment of a multifunction end effector including a rotational offset.

FIG. 18A illustrates the multifunction end effector of FIG. 17 in a closed configuration.

FIG. 18B illustrates the multifunction end effector of FIG. 17 in a first open configuration, in which access to a cutting portion is precluded.

FIG. 18C illustrates the multifunction end effector of FIG. 17 in a second open configuration that permits access to the cutting portion.

FIG. 18D illustrates a top view of the multifunction end effector of FIG. 18A in the closed configuration.

FIG. 18E illustrates a top view of the multifunction end effector of FIG. 18B in the first open configuration, in which access to a cutting portion is precluded.

FIG. 18F illustrates a top view of the multifunction end effector of FIG. 18C in the second open configuration that permits access to the cutting portion.

FIG. 19 illustrates example angles and dimensions of a multifunction end effector according to one embodiment.

FIG. 20A is a block diagram illustrating a robotically-enabled medical system including a multifunction end effector with a rotational offset.

FIG. 20B is a flowchart illustrating a method for implementing a multifunction end effector with rotational offsets.

FIG. 21A illustrates an embodiment of a multifunction end effector with a rotational offset performing a suturing procedure in a first mode that precludes access to a cutting portion.

FIG. 21B illustrates the multifunction end effector of FIG. 21A in a second mode that permits access to the cutting portion.

FIG. 22 illustrates an embodiment of a body for a multifunction end effector that includes a protrusion separating a gripping portion from a cutting portion.

FIG. 23 illustrates an alternative embodiment of a multifunction end effector.

## DETAILED **DESCRIPTION**

### 1. Overview.

Aspects of the present disclosure may be integrated into a robotically-enabled medical system capable of performing a variety of medical procedures, including both minimally invasive, such as laparoscopy, and non-invasive, such as endoscopy, procedures. Among endoscopy procedures, the system may be capable of performing bronchoscopy, ureteroscopy, gastroscopy, etc.

In addition to performing the breadth of procedures, the system may provide additional benefits, such as enhanced imaging and guidance to assist the physician. Additionally, the system may provide the physician with the ability to perform the procedure from an ergonomic position without the need for awkward arm motions and positions. Still further, the system may provide the physician with the ability to perform the procedure with improved ease of use such that one or more of the instruments of the system can be controlled by a single user.

Various embodiments will be described below in conjunction with the drawings for purposes of illustration. It should be appreciated that many other implementations of the disclosed concepts are possible, and various advantages can be achieved with the disclosed implementations. Headings are included herein for reference and to aid in locating various sections. These headings are not intended to limit the scope of the concepts described with respect thereto. Such concepts may have applicability throughout the entire specification.

### A. Robotic System - Cart.

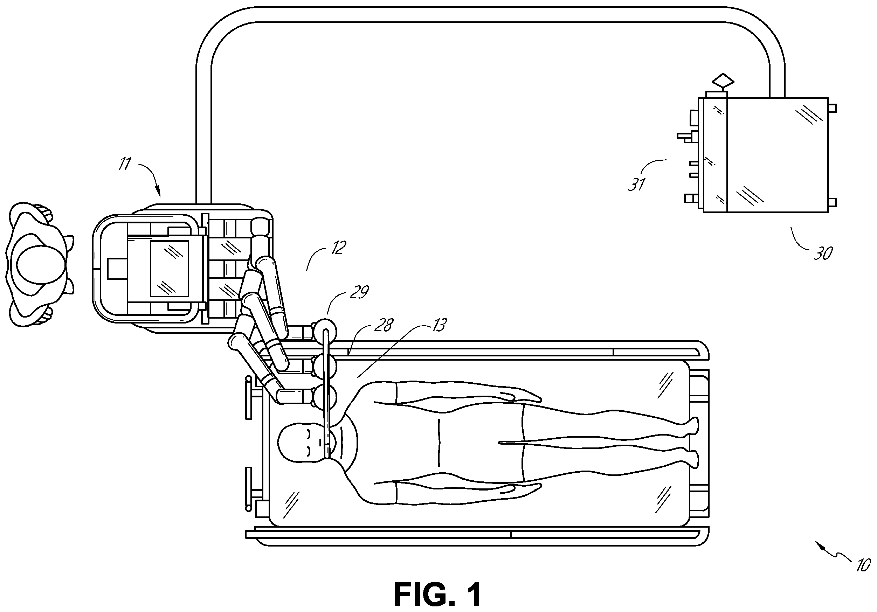

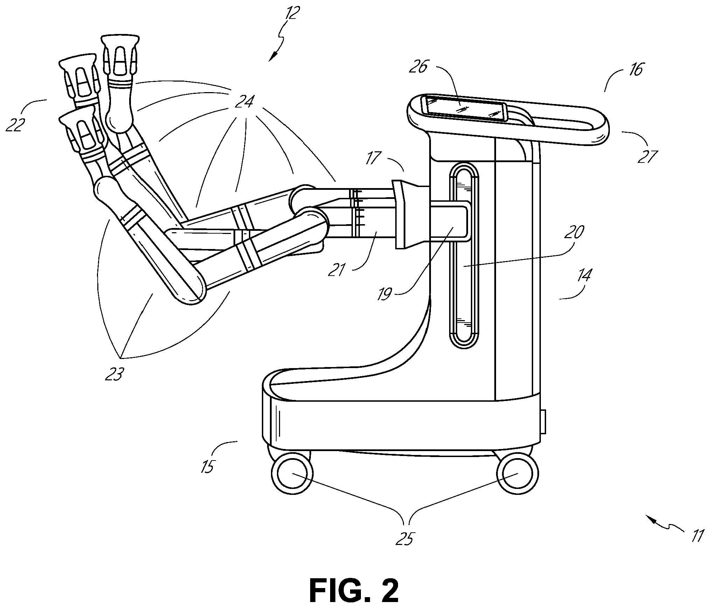

The robotically-enabled medical system may be configured in a variety of ways depending on the particular procedure. FIG. 1 illustrates an embodiment of a cart-based robotically-enabled system 10 arranged for a diagnostic and/or therapeutic bronchoscopy procedure. During a bronchoscopy, the system 10 may comprise a cart 11 having one or more robotic arms 12 to deliver a medical instrument, such as a steerable endoscope 13, which may be a procedure-specific bronchoscope for bronchoscopy, to a natural orifice access point (i.e., the mouth of the patient positioned on a table in the present example) to deliver diagnostic and/or therapeutic tools. As shown, the cart 11 may be positioned proximate to the patient's upper torso in order to provide access to the access point. Similarly, the robotic arms 12 may be actuated to position the bronchoscope relative to the access point. The arrangement in FIG. 1 may also be utilized when performing a gastro-intestinal (GI) procedure with a gastroscope, a specialized endoscope for GI procedures. FIG. 2 depicts an example embodiment of the cart in greater detail.

With continued reference to FIG. 1, once the cart 11 is properly positioned, the robotic arms 12 may insert the steerable endoscope 13 into the patient robotically, manually, or a combination thereof. As shown, the steerable endoscope 13 may comprise at least two telescoping parts, such as an inner leader portion and an outer sheath portion, each portion coupled to a separate instrument driver from the set of instrument drivers 28, each instrument driver coupled to the distal end of an individual robotic arm. This linear arrangement of the instrument drivers 28, which facilitates coaxially aligning the leader portion with the sheath portion, creates a "virtual rail" 29 that may be repositioned in space by manipulating the one or more robotic arms 12 into different angles and/or positions. The virtual rails described herein are depicted in the Figures using dashed lines, and accordingly the dashed lines do not depict any physical structure of the system. Translation of the instrument drivers 28 along the virtual rail 29 telescopes the inner leader portion relative to the outer sheath portion or advances or retracts the endoscope 13 from the patient. The angle of the virtual rail 29 may be adjusted, translated, and pivoted based on clinical application or physician preference. For example, in bronchoscopy, the angle and position of the virtual rail 29 as shown represents a compromise between providing physician access to the endoscope 13 while minimizing friction that results from bending the endoscope 13 into the patient's mouth.

The endoscope 13 may be directed down the patient's trachea and lungs after insertion using precise commands from the robotic system until reaching the target destination or operative site. In order to enhance navigation through the patient's lung network and/or reach the desired target, the endoscope 13 may be manipulated to telescopically extend the inner leader portion from the outer sheath portion to obtain enhanced articulation and greater bend radius. The use of separate instrument drivers 28 also allows the leader portion and sheath portion to be driven independent of each other.

For example, the endoscope 13 may be directed to deliver a biopsy needle to a target, such as, for example, a lesion or nodule within the lungs of a patient. The needle may be deployed down a working channel that runs the length of the endoscope to obtain a tissue sample to be analyzed by a pathologist. Depending on the pathology results, additional tools may be deployed down the working channel of the endoscope for additional biopsies. After identifying a nodule to be malignant, the endoscope 13 may endoscopically deliver tools to resect the potentially cancerous tissue. In some instances, diagnostic and therapeutic treatments may need to be delivered in separate procedures. In those circumstances, the endoscope 13 may also be used to deliver a fiducial to "mark" the location of the target nodule as well. In other instances, diagnostic and therapeutic treatments may be delivered during the same procedure.

The system 10 may also include a movable tower 30, which may be connected via support cables to the cart 11 to provide support for controls, electronics, fluidics, optics, sensors, and/or power to the cart 11. Placing such functionality in the tower 30 allows for a smaller form factor cart 11 that may be more easily adjusted and/or re-positioned by an operating physician and his/her staff. Additionally, the division of functionality between the cart / table and the support tower 30 reduces operating room clutter and facilitates improving clinical workflow. While the cart 11 may be positioned close to the patient, the tower 30 may be stowed in a remote location to stay out of the way during a procedure.

In support of the robotic systems described above, the tower 30 may include component(s) of a computer-based control system that stores computer program instructions, for example, within a non-transitory computer-readable storage medium such as a persistent magnetic storage drive, solid state drive, etc. The execution of those instructions, whether the execution occurs in the tower 30 or the cart 11, may control the entire system or sub-system(s) thereof. For example, when executed by a processor of the computer system, the instructions may cause the components of the robotics system to actuate the relevant carriages and arm mounts, actuate the robotics arms, and control the medical instruments. For example, in response to receiving the control signal, the motors in the joints of the robotics arms may position the arms into a certain posture.

The tower 30 may also include a pump, flow meter, valve control, and/or fluid access in order to provide controlled irrigation and aspiration capabilities to system that may be deployed through the endoscope 13. These components may also be controlled using the computer system of tower 30. In some embodiments, irrigation and aspiration capabilities may be delivered directly to the endoscope 13 through separate cable(s).

The tower 30 may include a voltage and surge protector designed to provide filtered and protected electrical power to the cart 11, thereby avoiding placement of a power transformer and other auxiliary power components in the cart 11, resulting in a smaller, more moveable cart 11.

The tower 30 may also include support equipment for the sensors deployed throughout the robotic system 10. For example, the tower 30 may include opto-electronics equipment for detecting, receiving, and processing data received from the optical sensors or cameras throughout the robotic system 10. In combination with the control system, such opto-electronics equipment may be used to generate real-time images for display in any number of consoles deployed throughout the system, including in the tower 30. Similarly, the tower 30 may also include an electronic subsystem for receiving and processing signals received from deployed electromagnetic (EM) sensors. The tower 30 may also be used to house and position an EM field generator for detection by EM sensors in or on the medical instrument.

The tower 30 may also include a console 31 in addition to other consoles available in the rest of the system, e.g., console mounted on top of the cart. The console 31 may include a user interface and a display screen, such as a touchscreen, for the physician operator. Consoles in system 10 are generally designed to provide both robotic controls as well as preoperative and real-time information of the procedure, such as navigational and localization information of the endoscope 13. When the console 31 is not the only console available to the physician, it may be used by a second operator, such as a nurse, to monitor the health or vitals of the patient and the operation of system, as well as provide procedure-specific data, such as navigational and localization information. In other embodiments, the console 30 is housed in a body that is separate from the tower 30.

The tower 30 may be coupled to the cart 11 and endoscope 13 through one or more cables or connections (not shown). In some embodiments, the support functionality from the tower 30 may be provided through a single cable to the cart 11, simplifying and de-cluttering the operating room. In other embodiments, specific functionality may be coupled in separate cabling and connections. For example, while power may be provided through a single power cable to the cart, the support for controls, optics, fluidics, and/or navigation may be provided through a separate cable.

FIG. 2 provides a detailed illustration of an embodiment of the cart from the cart-based robotically-enabled system shown in FIG. 1. The cart 11 generally includes an elongated support structure 14 (often referred to as a "column"), a cart base 15, and a console 16 at the top of the column 14. The column 14 may include one or more carriages, such as a carriage 17 (alternatively "arm support") for supporting the deployment of one or more robotic arms 12 (three shown in FIG. 2). The carriage 17 may include individually configurable arm mounts that rotate along a perpendicular axis to adjust the base of the robotic arms 12 for better positioning relative to the patient. The carriage 17 also includes a carriage interface 19 that allows the carriage 17 to vertically translate along the column 14.

The carriage interface 19 is connected to the column 14 through slots, such as slot 20, that are positioned on opposite sides of the column 14 to guide the vertical translation of the carriage 17. The slot 20 contains a vertical translation interface to position and hold the carriage at various vertical heights relative to the cart base 15. Vertical translation of the carriage 17 allows the cart 11 to adjust the reach of the robotic arms 12 to meet a variety of table heights, patient sizes, and physician preferences. Similarly, the individually configurable arm mounts on the carriage 17 allow the robotic arm base 21 of robotic arms 12 to be angled in a variety of configurations.

In some embodiments, the slot 20 may be supplemented with slot covers that are flush and parallel to the slot surface to prevent dirt and fluid ingress into the internal chambers of the column 14 and the vertical translation interface as the carriage 17 vertically translates. The slot covers may be deployed through pairs of spring spools positioned near the vertical top and bottom of the slot 20. The covers are coiled within the spools until deployed to extend and retract from their coiled state as the carriage 17 vertically translates up and down. The spring-loading of the spools provides force to retract the cover into a spool when carriage 17 translates towards the spool, while also maintaining a tight seal when the carriage 17 translates away from the spool. The covers may be connected to the carriage 17 using, for example, brackets in the carriage interface 19 to ensure proper extension and retraction of the cover as the carriage 17 translates.

The column 14 may internally comprise mechanisms, such as gears and motors, that are designed to use a vertically aligned lead screw to translate the carriage 17 in a mechanized fashion in response to control signals generated in response to user inputs, e.g., inputs from the console 16.

The robotic arms 12 may generally comprise robotic arm bases 21 and end effectors 22, separated by a series of linkages 23 that are connected by a series of joints 24, each joint comprising an independent actuator, each actuator comprising an independently controllable motor. Each independently controllable joint represents an independent degree of freedom available to the robotic arm. Each of the arms 12 have seven joints, and thus provide seven degrees of freedom. A multitude of joints result in a multitude of degrees of freedom, allowing for "redundant" degrees of freedom. Redundant degrees of freedom allow the robotic arms 12 to position their respective end effectors 22 at a specific position, orientation, and trajectory in space using different linkage positions and joint angles. This allows for the system to position and direct a medical instrument from a desired point in space while allowing the physician to move the arm joints into a clinically advantageous position away from the patient to create greater access, while avoiding arm collisions.

The cart base 15 balances the weight of the column 14, carriage 17, and arms 12 over the floor. Accordingly, the cart base 15 houses heavier components, such as electronics, motors, power supply, as well as components that either enable movement and/or immobilize the cart. For example, the cart base 15 includes rollable wheel-shaped casters 25 that allow for the cart to easily move around the room prior to a procedure. After reaching the appropriate position, the casters 25 may be immobilized using wheel locks to hold the cart 11 in place during the procedure.

Positioned at the vertical end of column 14, the console 16 allows for both a user interface for receiving user input and a display screen (or a dual-purpose device such as, for example, a touchscreen 26) to provide the physician user with both pre-operative and intra-operative data. Potential pre-operative data on the touchscreen 26 may include pre-operative plans, navigation and mapping data derived from pre-operative computerized tomography (CT) scans, and/or notes from pre-operative patient interviews. Intra-operative data on display may include optical information provided from the tool, sensor and coordinate information from sensors, as well as vital patient statistics, such as respiration, heart rate, and/or pulse. The console 16 may be positioned and tilted to allow a physician to access the console from the side of the column 14 opposite carriage 17. From this position, the physician may view the console 16, robotic arms 12, and patient while operating the console 16 from behind the cart 11. As shown, the console 16 also includes a handle 27 to assist with maneuvering and stabilizing cart 11.

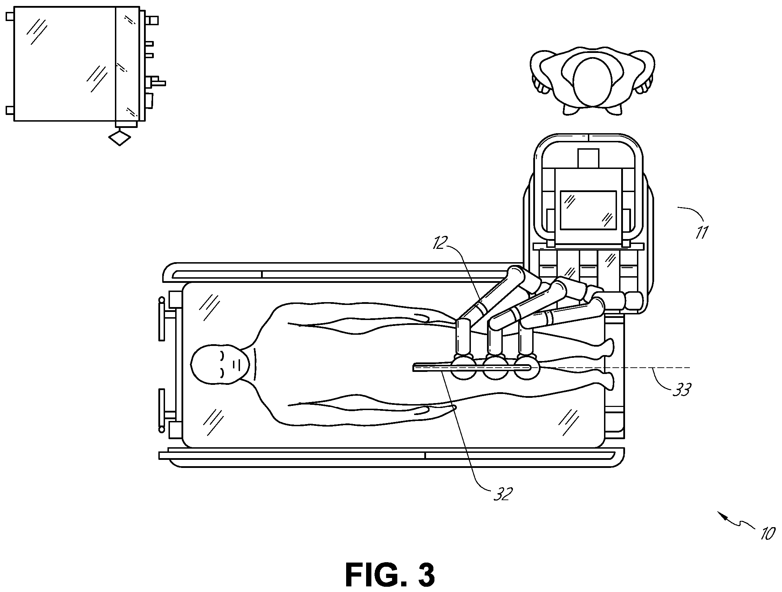

FIG. 3 illustrates an embodiment of a robotically-enabled system 10 arranged for ureteroscopy. In a ureteroscopic procedure, the cart 11 may be positioned to deliver a ureteroscope 32, a procedure-specific endoscope designed to traverse a patient's urethra and ureter, to the lower abdominal area of the patient. In a ureteroscopy, it may be desirable for the ureteroscope 32 to be directly aligned with the patient's urethra to reduce friction and forces on the sensitive anatomy in the area. As shown, the cart 11 may be aligned at the foot of the table to allow the robotic arms 12 to position the ureteroscope 32 for direct linear access to the patient's urethra. From the foot of the table, the robotic arms 12 may insert the ureteroscope 32 along the virtual rail 33 directly into the patient's lower abdomen through the urethra.

After insertion into the urethra, using similar control techniques as in bronchoscopy, the ureteroscope 32 may be navigated into the bladder, ureters, and/or kidneys for diagnostic and/or therapeutic applications. For example, the ureteroscope 32 may be directed into the ureter and kidneys to break up kidney stone build up using laser or ultrasonic lithotripsy device deployed down the working channel of the ureteroscope 32. After lithotripsy is complete, the resulting stone fragments may be removed using baskets deployed down the ureteroscope 32.

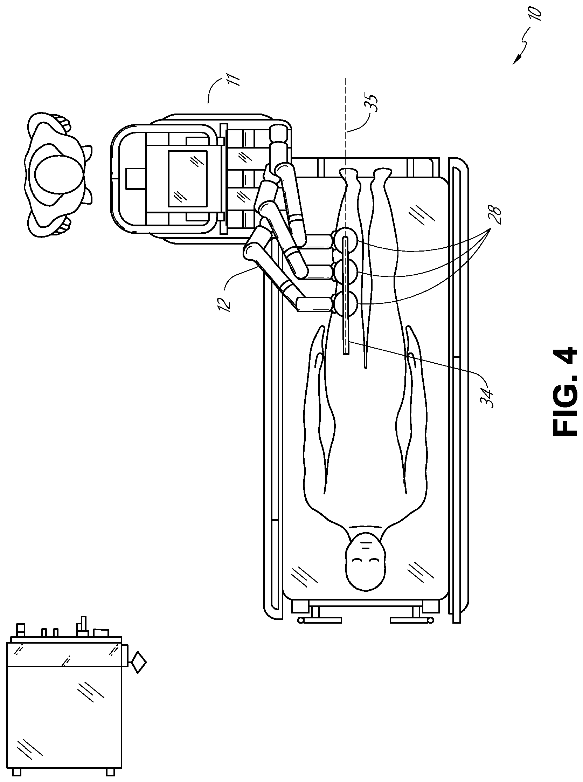

FIG. 4 illustrates an embodiment of a robotically-enabled system similarly arranged for a vascular procedure. In a vascular procedure, the system 10 may be configured such the cart 11 may deliver a medical instrument 34, such as a steerable catheter, to an access point in the femoral artery in the patient's leg. The femoral artery presents both a larger diameter for navigation as well as relatively less circuitous and tortuous path to the patient's heart, which simplifies navigation. As in a ureteroscopic procedure, the cart 11 may be positioned towards the patient's legs and lower abdomen to allow the robotic arms 12 to provide a virtual rail 35 with direct linear access to the femoral artery access point in the patient's thigh / hip region. After insertion into the artery, the medical instrument 34 may be directed and inserted by translating the instrument drivers 28. Alternatively, the cart may be positioned around the patient's upper abdomen in order to reach alternative vascular access points, such as, for example, the carotid and brachial arteries near the shoulder and wrist.

## B. Robotic System - Table.

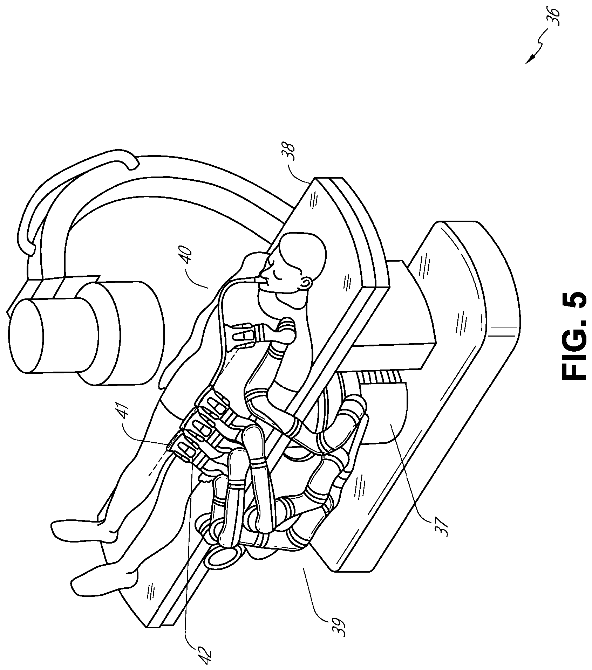

Embodiments of the robotically-enabled medical system may also incorporate the patient's table. Incorporation of the table reduces the amount of capital equipment within the operating room by removing the cart, which allows greater access to the patient. FIG. 5 illustrates an embodiment of such a robotically-enabled system arranged for a bronchoscopy procedure. System 36 includes a support structure or column 37 for supporting platform 38 (shown as a "table" or "bed") over the floor. Much like in the cart-based systems, the end effectors of the robotic arms 39 of the system 36 comprise instrument drivers 42 that are designed to manipulate an elongated medical instrument, such as a bronchoscope 40 in FIG. 5, through or along a virtual rail 41 formed from the linear alignment of the instrument drivers 42. In practice, a C-arm for providing fluoroscopic imaging may be positioned over the patient's upper abdominal area by placing the emitter and detector around table 38.

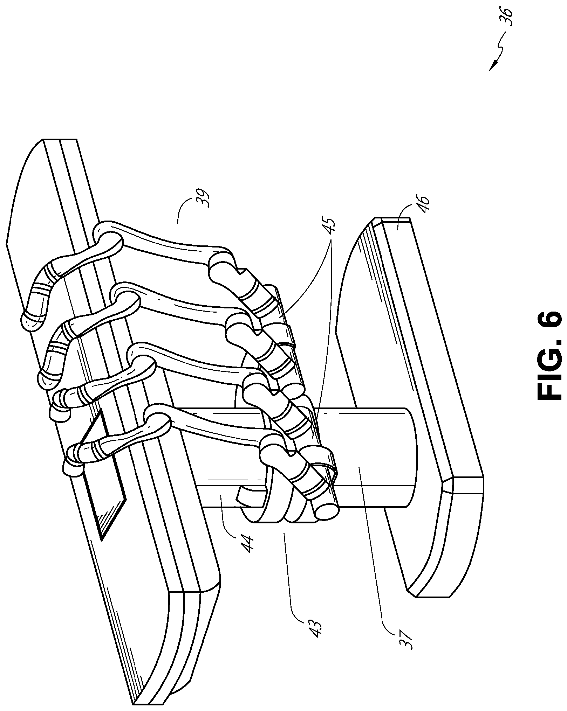

FIG. 6 provides an alternative view of the system 36 without the patient and medical instrument for discussion purposes. As shown, the column 37 may include one or more carriages 43 shown as ring-shaped in the system 36, from which the one or more robotic arms 39 may be based. The carriages 43 may translate along a vertical column interface 44 that runs the length of the column 37 to provide different vantage points from which the robotic arms 39 may be positioned to reach the patient. The carriage(s) 43 may rotate around the column 37 using a mechanical motor positioned within the column 3 7 to allow the robotic arms 39 to have access to multiples sides of the table 38, such as, for example, both sides of the patient. In embodiments with multiple carriages, the carriages may be individually positioned on the column and may translate and/or rotate independent of the other carriages. While carriages 43 need not surround the column 37 or even be circular, the ring-shape as shown facilitates rotation of the carriages 43 around the column 37 while maintaining structural balance. Rotation and translation of the carriages 43 allows the system to align the medical instruments, such as endoscopes and laparoscopes, into different access points on the patient. In other embodiments (not shown), the system 36 can include a patient table or bed with adjustable arm supports in the form of bars or rails extending alongside it. One or more robotic arms 39 (e.g., via a shoulder with an elbow joint) can be attached to the adjustable arm supports, which can be vertically adjusted. By providing vertical adjustment, the robotic arms 39 are advantageously capable of being stowed compactly beneath the patient table or bed, and subsequently raised during a procedure.

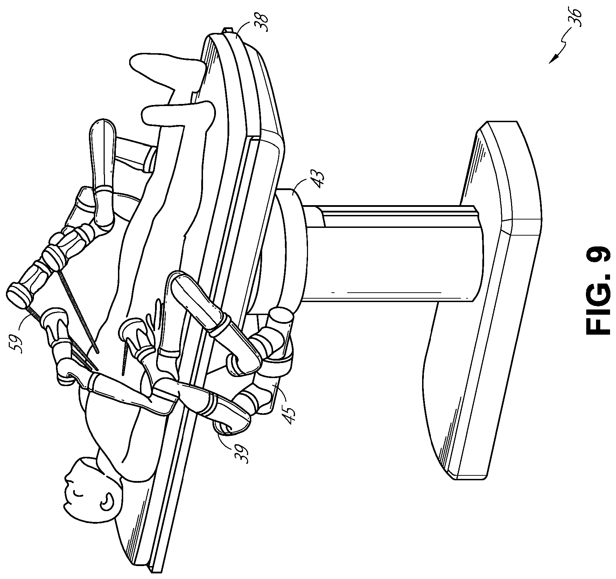

The arms 39 may be mounted on the carriages through a set of arm mounts 45 comprising a series of joints that may individually rotate and/or telescopically extend to provide additional configurability to the robotic arms 39. Additionally, the arm mounts 45 may be positioned on the carriages 43 such that, when the carriages 43 are appropriately rotated, the arm mounts 45 may be positioned on either the same side of table 38 (as shown in FIG. 6), on opposite sides of table 38 (as shown in FIG. 9), or on adjacent sides of the table 38 (not shown).

The column 37 structurally provides support for the table 38, and a path for vertical translation of the carriages. Internally, the column 37 may be equipped with lead screws for guiding vertical translation of the carriages, and motors to mechanize the translation of said carriages based the lead screws. The column 37 may also convey power and control signals to the carriage 43 and robotic arms 39 mounted thereon.

The table base 46 serves a similar function as the cart base 15 in cart 11 shown in FIG. 2, housing heavier components to balance the table/bed 38, the column 37, the carriages 43, and the robotic arms 39. The table base 46 may also incorporate rigid casters to provide stability during procedures. Deployed from the bottom of the table base 46, the casters may extend in opposite directions on both sides of the base 46 and retract when the system 36 needs to be moved.

Continuing with FIG. 6, the system 36 may also include a tower (not shown) that divides the functionality of system 36 between table and tower to reduce the form factor and bulk of the table. As in earlier disclosed embodiments, the tower may provide a variety of support functionalities to table, such as processing, computing, and control capabilities, power, fluidics, and/or optical and sensor processing. The tower may also be movable to be positioned away from the patient to improve physician access and de-clutter the operating room. Additionally, placing components in the tower allows for more storage space in the table base for potential stowage of the robotic arms. The tower may also include a master controller or console that provides both a user interface for user input, such as keyboard and/or pendant, as well as a display screen (or touchscreen) for pre-operative and intra-operative information, such as real-time imaging, navigation, and tracking information. In some embodiments, the tower may also contain holders for gas tanks to be used for insufflation.

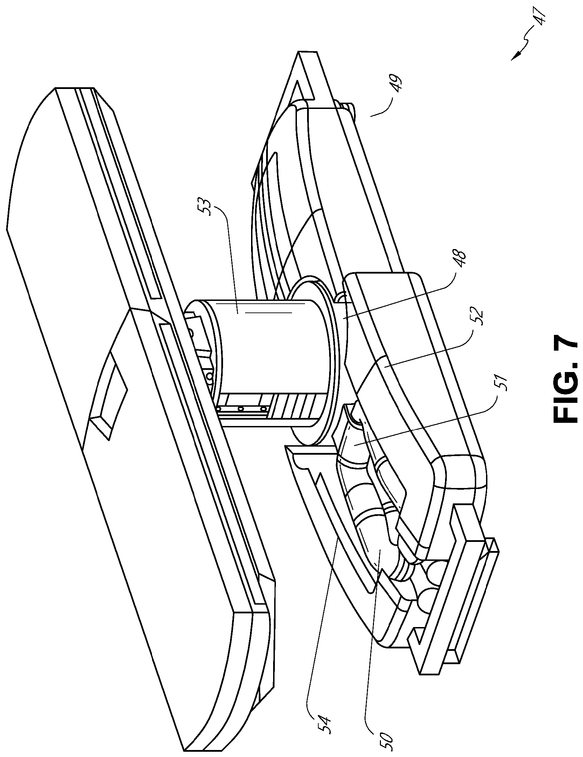

In some embodiments, a table base may stow and store the robotic arms when not in use. FIG. 7 illustrates a system 47 that stows robotic arms in an embodiment of the table-based system. In system 47, carriages 48 may be vertically translated into base 49 to stow robotic arms 50, arm mounts 51, and the carriages 48 within the base 49. Base covers 52 may be translated and retracted open to deploy the carriages 48, arm mounts 51, and arms 50 around column 53, and closed to stow to protect them when not in use. The base covers 52 may be sealed with a membrane 54 along the edges of its opening to prevent dirt and fluid ingress when closed.

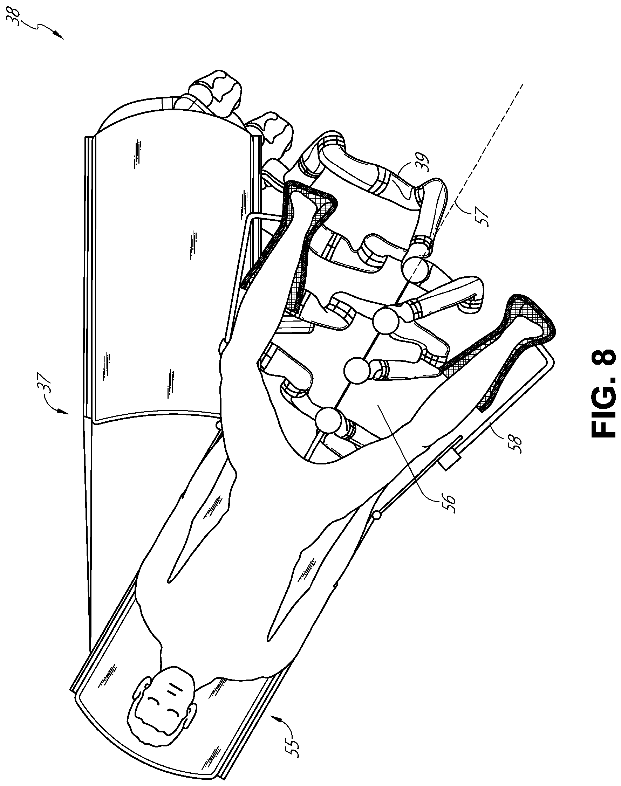

FIG. 8 illustrates an embodiment of a robotically-enabled table-based system configured for a ureteroscopy procedure. In a ureteroscopy, the table 38 may include a swivel portion 55 for positioning a patient off-angle from the column 37 and table base 46. The swivel portion 55 may rotate or pivot around a pivot point (e.g., located below the patient's head) in order to position the bottom portion of the swivel portion 55 away from the column 37. For example, the pivoting of the swivel portion 55 allows a C-arm (not shown) to be positioned over the patient's lower abdomen without competing for space with the column (not shown) below table 38. By rotating the carriage 35 (not shown) around the column 37, the robotic arms 39 may directly insert a ureteroscope 56 along a virtual rail 57 into the patient's groin area to reach the urethra. In a ureteroscopy, stirrups 58 may also be fixed to the swivel portion 55 of the table 38 to support the position of the patient's legs during the procedure and allow clear access to the patient's groin area.

In a laparoscopic procedure, through small incision(s) in the patient's abdominal wall, minimally invasive instruments may be inserted into the patient's anatomy. In some embodiments, the minimally invasive instruments comprise an elongated rigid member, such as a shaft, which is used to access anatomy within the patient. After inflation of the patient's abdominal cavity, the instruments may be directed to perform surgical or medical tasks, such as grasping, cutting, ablating, suturing, etc. In some embodiments, the instruments can comprise a scope, such as a laparoscope. FIG. 9 illustrates an embodiment of a robotically-enabled table-based system configured for a laparoscopic procedure. As shown in FIG. 9, the carriages 43 of the system 36 may be rotated and vertically adjusted to position pairs of the robotic arms 39 on opposite sides of the table 38, such that instrument 59 may be positioned using the arm mounts 45 to be passed through minimal incisions on both sides of the patient to reach his/her abdominal cavity.

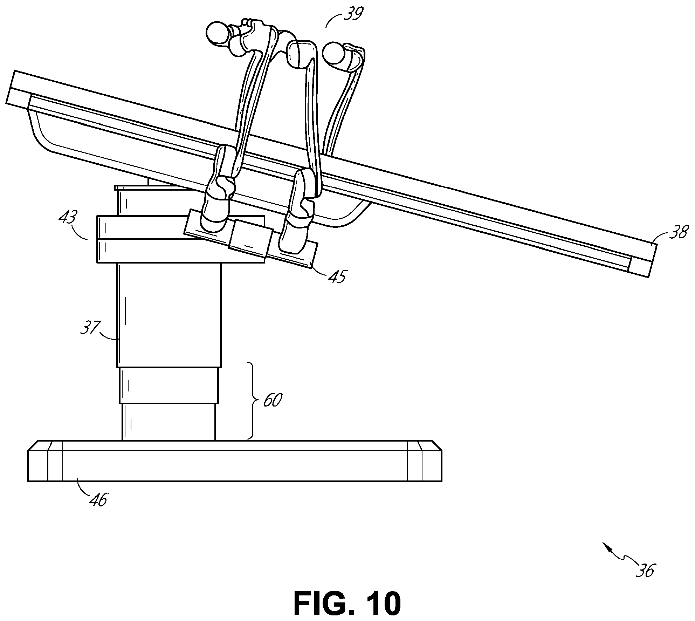

To accommodate laparoscopic procedures, the robotically-enabled table system may also tilt the platform to a desired angle. FIG. 10 illustrates an embodiment of the robotically-enabled medical system with pitch or tilt adjustment. As shown in FIG. 10, the system 36 may accommodate tilt of the table 38 to position one portion of the table at a greater distance from the floor than the other. Additionally, the arm mounts 45 may rotate to match the tilt such that the arms 39 maintain the same planar relationship with table 38. To accommodate steeper angles, the column 37 may also include telescoping portions 60 that allow vertical extension of column 37 to keep the table 38 from touching the floor or colliding with base 46.

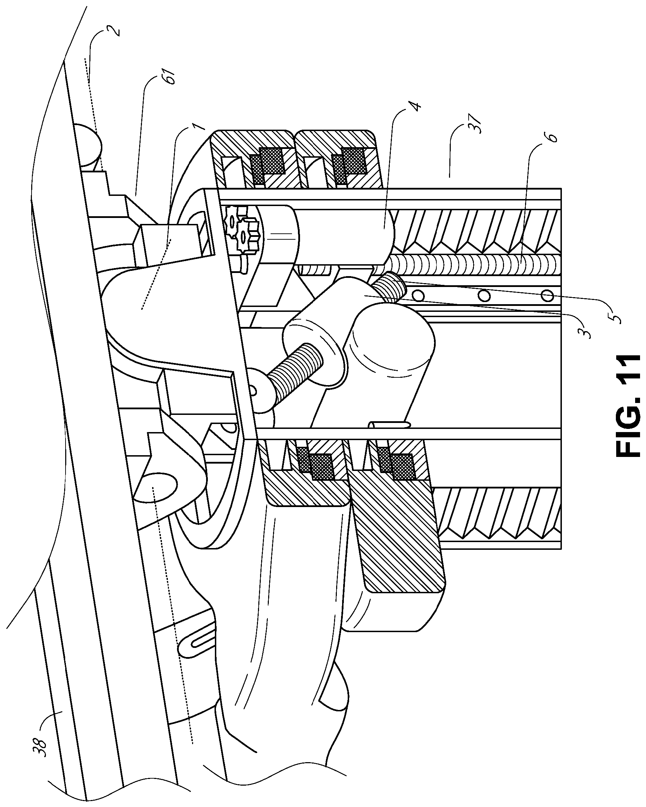

FIG. 11 provides a detailed illustration of the interface between the table 38 and the column 37. Pitch rotation mechanism 61 may be configured to alter the pitch angle of the table 38 relative to the column 37 in multiple degrees of freedom. The pitch rotation mechanism 61 may be enabled by the positioning of orthogonal axes 1, 2 at the column-table interface, each axis actuated by a separate motor 3, 4 responsive to an electrical pitch angle command. Rotation along one screw 5 would enable tilt adjustments in one axis 1, while rotation along the other screw 6 would enable tilt adjustments along the other axis 2. In some embodiments, a ball joint can be used to alter the pitch angle of the table 38 relative to the column 3 7 in multiple degrees of freedom.

For example, pitch adjustments are particularly useful when trying to position the table in a Trendelenburg position, i.e., position the patient's lower abdomen at a higher position from the floor than the patient's lower abdomen, for lower abdominal surgery. The Trendelenburg position causes the patient's internal organs to slide towards his/her upper abdomen through the force of gravity, clearing out the abdominal cavity for minimally invasive tools to enter and perform lower abdominal surgical or medical procedures, such as laparoscopic prostatectomy.

## C. Instrument Driver & Interface.

The end effectors of the system's robotic arms comprise (i) an instrument driver (alternatively referred to as "instrument drive mechanism" or "instrument device manipulator") that incorporate electro-mechanical means for actuating the medical instrument and (ii) a removable or detachable medical instrument, which may be devoid of any electro-mechanical components, such as motors. This dichotomy may be driven by the need to sterilize medical instruments used in medical procedures, and the inability to adequately sterilize expensive capital equipment due to their intricate mechanical assemblies and sensitive electronics. Accordingly, the medical instruments may be designed to be detached, removed, and interchanged from the instrument driver (and thus the system) for individual sterilization or disposal by the physician or the physician's staff. In contrast, the instrument drivers need not be changed or sterilized, and may be draped for protection.

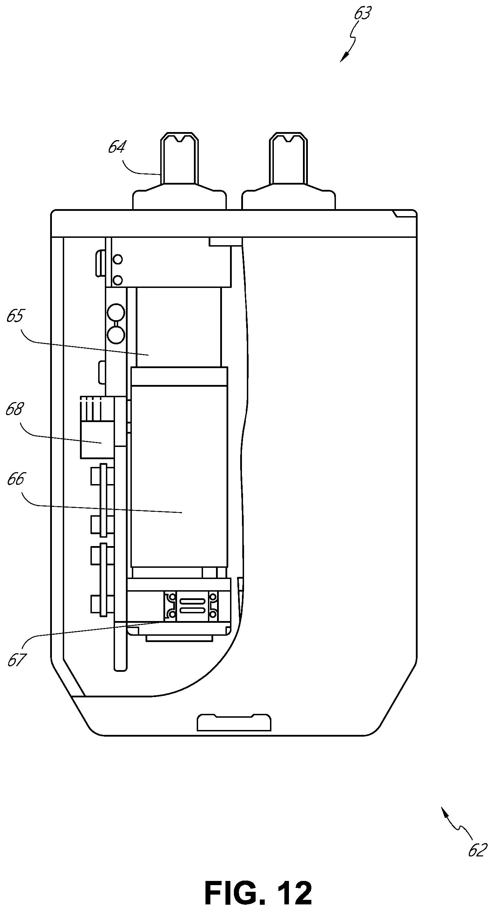

FIG. 12 illustrates an example instrument driver. Positioned at the distal end of a robotic arm, instrument driver 62 comprises of one or more drive units 63 arranged with parallel axes to provide controlled torque to a medical instrument via drive shafts 64. Each drive unit 63 comprises an individual drive shaft 64 for interacting with the instrument, a gear head 65 for converting the motor shaft rotation to a desired torque, a motor 66 for generating the drive torque, an encoder 67 to measure the speed of the motor shaft and provide feedback to the control circuitry, and control circuity 68 for receiving control signals and actuating the drive unit. Each drive unit 63 being independent controlled and motorized, the instrument driver 62 may provide multiple (four as shown in FIG. 12) independent drive outputs to the medical instrument. In operation, the control circuitry 68 would receive a control signal, transmit a motor signal to the motor 66, compare the resulting motor speed as measured by the encoder 67 with the desired speed, and modulate the motor signal to generate the desired torque.

For procedures that require a sterile environment, the robotic system may incorporate a drive interface, such as a sterile adapter connected to a sterile drape, that sits between the instrument driver and the medical instrument. The chief purpose of the sterile adapter is to transfer angular motion from the drive shafts of the instrument driver to the drive inputs of the instrument while maintaining physical separation, and thus sterility, between the drive shafts and drive inputs. Accordingly, an example sterile adapter may comprise of a series of rotational inputs and outputs intended to be mated with the drive shafts of the instrument driver and drive inputs on the instrument. Connected to the sterile adapter, the sterile drape, comprised of a thin, flexible material such as transparent or translucent plastic, is designed to cover the capital equipment, such as the instrument driver, robotic arm, and cart (in a cart-based system) or table (in a table-based system). Use of the drape would allow the capital equipment to be positioned proximate to the patient while still being located in an area not requiring sterilization (i.e., non-sterile field). On the other side of the sterile drape, the medical instrument may interface with the patient in an area requiring sterilization (i.e., sterile field).

### D. Medical Instrument.

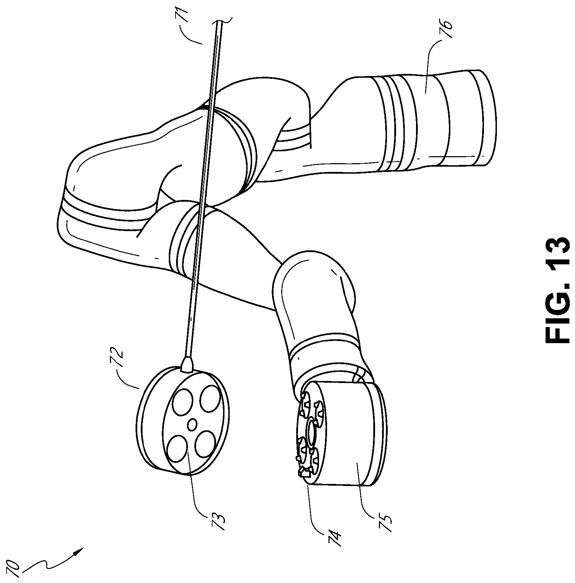

FIG. 13 illustrates an example medical instrument with a paired instrument driver. Like other instruments designed for use with a robotic system, medical instrument 70 comprises an elongated shaft 71 (or elongate body) and an instrument base 72. The instrument base 72, also referred to as an "instrument handle" due to its intended design for manual interaction by the physician, may generally comprise rotatable drive inputs 73, e.g., receptacles, pulleys or spools, that are designed to be mated with drive outputs 74 that extend through a drive interface on instrument driver 75 at the distal end of robotic arm 76. When physically connected, latched, and/or coupled, the mated drive inputs 73 of instrument base 72 may share axes of rotation with the drive outputs 74 in the instrument driver 75 to allow the transfer of torque from drive outputs 74 to drive inputs 73. In some embodiments, the drive outputs 74 may comprise splines that are designed to mate with receptacles on the drive inputs 73.

The elongated shaft 71 is designed to be delivered through either an anatomical opening or lumen, e.g., as in endoscopy, or a minimally invasive incision, e.g., as in laparoscopy. The elongated shaft 71 may be either flexible (e.g., having properties similar to an endoscope) or rigid (e.g., having properties similar to a laparoscope) or contain a customized combination of both flexible and rigid portions. When designed for laparoscopy, the distal end of a rigid elongated shaft may be connected to an end effector extending from a jointed wrist formed from a clevis with at least one degree of freedom and a surgical tool or medical instrument, such as, for example, a grasper or scissors, that may be actuated based on force from the tendons as the drive inputs rotate in response to torque received from the drive outputs 74 of the instrument driver 75. When designed for endoscopy, the distal end of a flexible elongated shaft may include a steerable or controllable bending section that may be articulated and bent based on torque received from the drive outputs 74 of the instrument driver 75.

Torque from the instrument driver 75 is transmitted down the elongated shaft 71 using tendons along the shaft 71. These individual tendons, such as pull wires, may be individually anchored to individual drive inputs 73 within the instrument handle 72. From the handle 72, the tendons are directed down one or more pull lumens along the elongated shaft 71 and anchored at the distal portion of the elongated shaft 71, or in the wrist at the distal portion of the elongated shaft. During a surgical procedure, such as a laparoscopic, endoscopic or hybrid procedure, these tendons may be coupled to a distally mounted end effector, such as a wrist, grasper, or scissor. Under such an arrangement, torque exerted on drive inputs 73 would transfer tension to the tendon, thereby causing the end effector to actuate in some way. In some embodiments, during a surgical procedure, the tendon may cause a joint to rotate about an axis, thereby causing the end effector to move in one direction or another. Alternatively, the tendon may be connected to one or more jaws of a grasper at distal end of the elongated shaft 71, where tension from the tendon cause the grasper to close.

In endoscopy, the tendons may be coupled to a bending or articulating section positioned along the elongated shaft 71 (e.g., at the distal end) via adhesive, control ring, or other mechanical fixation. When fixedly attached to the distal end of a bending section, torque exerted on drive inputs 73 would be transmitted down the tendons, causing the softer, bending section (sometimes referred to as the articulable section or region) to bend or articulate. Along the nonbending sections, it may be advantageous to spiral or helix the individual pull lumens that direct the individual tendons along (or inside) the walls of the endoscope shaft to balance the radial forces that result from tension in the pull wires. The angle of the spiraling and/or spacing there between may be altered or engineered for specific purposes, wherein tighter spiraling exhibits lesser shaft compression under load forces, while lower amounts of spiraling results in greater shaft compression under load forces, but also exhibits limits bending. On the other end of the spectrum, the pull lumens may be directed parallel to the longitudinal axis of the elongated shaft 71 to allow for controlled articulation in the desired bending or articulable sections.

In endoscopy, the elongated shaft 71 houses a number of components to assist with the robotic procedure. The shaft may comprise of a working channel for deploying surgical tools (or medical instruments), irrigation, and/or aspiration to the operative region at the distal end of the shaft 71. The shaft 71 may also accommodate wires and/or optical fibers to transfer signals to/from an optical assembly at the distal tip, which may include of an optical camera. The shaft 71 may also accommodate optical fibers to carry light from proximally-located light sources, such as light emitting diodes, to the distal end of the shaft.

At the distal end of the instrument 70, the distal tip may also comprise the opening of a working channel for delivering tools for diagnostic and/or therapy, irrigation, and aspiration to an operative site. The distal tip may also include a port for a camera, such as a fiberscope or a digital camera, to capture images of an internal anatomical space. Relatedly, the distal tip may also include ports for light sources for illuminating the anatomical space when using the camera.

In the example of FIG. 13, the drive shaft axes, and thus the drive input axes, are orthogonal to the axis of the elongated shaft. This arrangement, however, complicates roll capabilities for the elongated shaft 71. Rolling the elongated shaft 71 along its axis while keeping the drive inputs 73 static results in undesirable tangling of the tendons as they extend off the drive inputs 73 and enter pull lumens within the elongated shaft 71. The resulting entanglement of such tendons may disrupt any control algorithms intended to predict movement of the flexible elongated shaft during an endoscopic procedure.

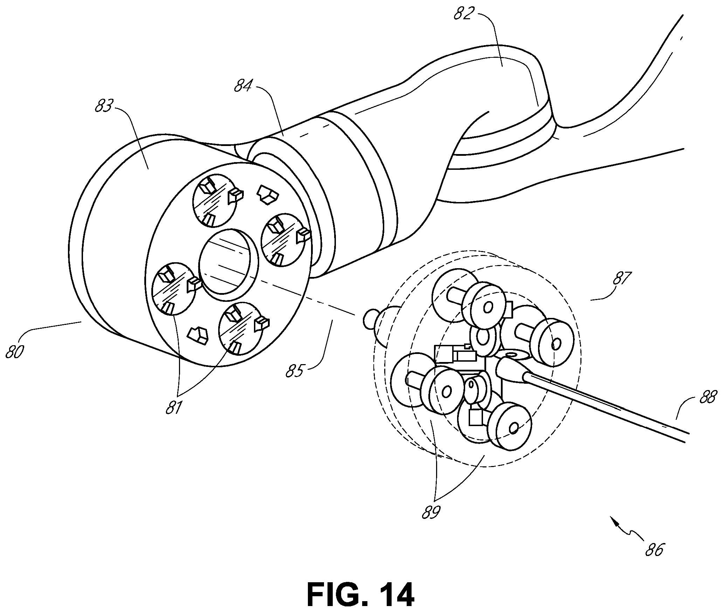

FIG. 14 illustrates an alternative design for an instrument driver and instrument where the axes of the drive units are parallel to the axis of the elongated shaft of the instrument. As shown, a circular instrument driver 80 comprises four drive units with their drive outputs 81 aligned in parallel at the end of a robotic arm 82. The drive units, and their respective drive outputs 81, are housed in a rotational assembly 83 of the instrument driver 80 that is driven by one of the drive units within the assembly 83. In response to torque provided by the rotational drive unit, the rotational assembly 83 rotates along a circular bearing that connects the rotational assembly 83 to the non-rotational portion 84 of the instrument driver. Power and controls signals may be communicated from the non-rotational portion 84 of the instrument driver 80 to the rotational assembly 83 through electrical contacts may be maintained through rotation by a brushed slip ring connection (not shown). In other embodiments, the rotational assembly 83 may be responsive to a separate drive unit that is integrated into the non-rotatable portion 84, and thus not in parallel to the other drive units. The rotational mechanism 83 allows the instrument driver 80 to rotate the drive units, and their respective drive outputs 81, as a single unit around an instrument driver axis 85.

Like earlier disclosed embodiments, an instrument 86 may comprise an elongated shaft portion 88 and an instrument base 87 (shown with a transparent external skin for discussion purposes) comprising a plurality of drive inputs 89 (such as receptacles, pulleys, and spools) that are configured to receive the drive outputs 81 in the instrument driver 80. Unlike prior disclosed embodiments, instrument shaft 88 extends from the center of instrument base 87 with an axis substantially parallel to the axes of the drive inputs 89, rather than orthogonal as in the design of FIG. 13.

When coupled to the rotational assembly 83 of the instrument driver 80, the medical instrument 86, comprising instrument base 87 and instrument shaft 88, rotates in combination with the rotational assembly 83 about the instrument driver axis 85. Since the instrument shaft 88 is positioned at the center of instrument base 87, the instrument shaft 88 is coaxial with instrument driver axis 85 when attached. Thus, rotation of the rotational assembly 83 causes the instrument shaft 88 to rotate about its own longitudinal axis. Moreover, as the instrument base 87 rotates with the instrument shaft 88, any tendons connected to the drive inputs 89 in the instrument base 87 are not tangled during rotation. Accordingly, the parallelism of the axes of the drive outputs 81, drive inputs 89, and instrument shaft 88 allows for the shaft rotation without tangling any control tendons.

### E. Navigation and Control.

Traditional endoscopy may involve the use of fluoroscopy (e.g., as may be delivered through a C-arm) and other forms of radiation-based imaging modalities to provide endoluminal guidance to an operator physician. In contrast, the robotic systems contemplated by this disclosure can provide for non-radiation-based navigational and localization means to reduce physician exposure to radiation and reduce the amount of equipment within the operating room. As used herein, the term "localization" may refer to determining and/or monitoring the position of objects in a reference coordinate system. Technologies such as pre-operative mapping, computer vision, real-time EM tracking, and robot command data may be used individually or in combination to achieve a radiation-free operating environment. In other cases, where radiation-based imaging modalities are still used, the pre-operative mapping, computer vision, real-time EM tracking, and robot command data may be used individually or in combination to improve upon the information obtained solely through radiation-based imaging modalities.

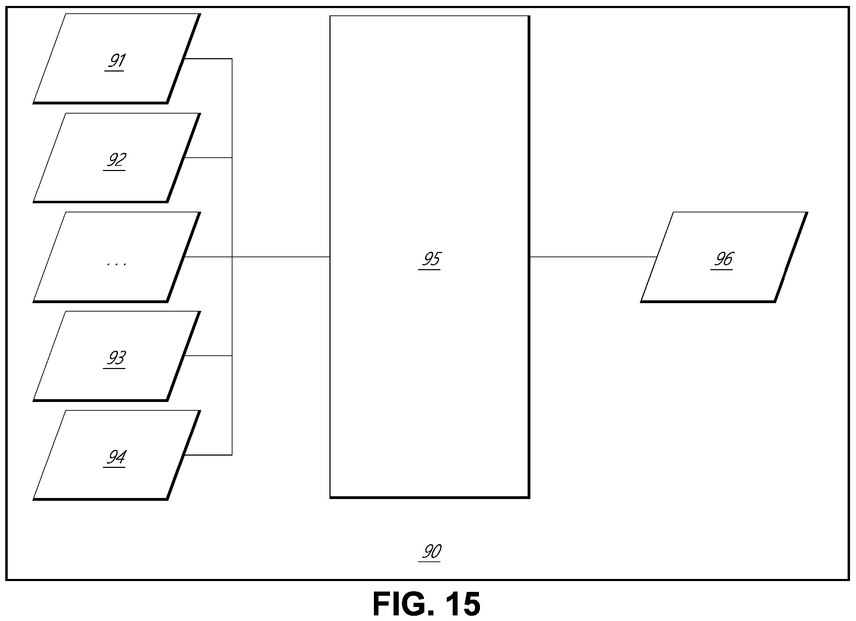

FIG. 15 is a block diagram illustrating a localization system 90 that estimates a location of one or more elements of the robotic system, such as the location of the instrument, in accordance to an example embodiment. The localization system 90 may be a set of one or more computer devices configured to execute one or more instructions. The computer devices may be embodied by a processor (or processors) and computer-readable memory in one or more components discussed above. By way of example and not limitation, the computer devices may be in the tower 30 shown in FIG. 1, the cart shown in FIGs. 1-4, the beds shown in FIGs. 5-10, etc.

As shown in FIG. 15, the localization system 90 may include a localization module 95 that processes input data 91-94 to generate location data 96 for the distal tip of a medical instrument. The location data 96 may be data or logic that represents a location and/or orientation of the distal end of the instrument relative to a frame of reference. The frame of reference can be a frame of reference relative to the anatomy of the patient or to a known object, such as an EM field generator (see discussion below for the EM field generator).

The various input data 91-94 are now described in greater detail. Pre-operative mapping may be accomplished through the use of the collection of low dose CT scans. Preoperative CT scans are reconstructed into three-dimensional images, which are visualized, e.g. as "slices" of a cutaway view of the patient's internal anatomy. When analyzed in the aggregate, image-based models for anatomical cavities, spaces and structures of the patient's anatomy, such as a patient lung network, may be generated. Techniques such as center-line geometry may be determined and approximated from the CT images to develop a three-dimensional volume of the patient's anatomy, referred to as model data 91 (also referred to as "preoperative model data" when generated using only preoperative CT scans). The use of center-line geometry is discussed in U.S. Pat. App. No. 14/523,760. Network topological models may also be derived from the CT-images, and are particularly appropriate for bronchoscopy.

In some embodiments, the instrument may be equipped with a camera to provide vision data 92. The localization module 95 may process the vision data to enable one or more vision-based location tracking. For example, the preoperative model data may be used in conjunction with the vision data 92 to enable computer vision-based tracking of the medical instrument (e.g., an endoscope or an instrument advance through a working channel of the endoscope). For example, using the preoperative model data 91, the robotic system may generate a library of expected endoscopic images from the model based on the expected path of travel of the endoscope, each image linked to a location within the model. Intra-operatively, this library may be referenced by the robotic system in order to compare real-time images captured at the camera (e.g., a camera at a distal end of the endoscope) to those in the image library to assist localization.

Other computer vision-based tracking techniques use feature tracking to determine motion of the camera, and thus the endoscope. Some features of the localization module 95 may identify circular geometries in the preoperative model data 91 that correspond to anatomical lumens and track the change of those geometries to determine which anatomical lumen was selected, as well as the relative rotational and/or translational motion of the camera. Use of a topological map may further enhance vision-based algorithms or techniques.

Optical flow, another computer vision-based technique, may analyze the displacement and translation of image pixels in a video sequence in the vision data 92 to infer camera movement. Examples of optical flow techniques may include motion detection, object segmentation calculations, luminance, motion compensated encoding, stereo disparity measurement, etc. Through the comparison of multiple frames over multiple iterations, movement and location of the camera (and thus the endoscope) may be determined.

The localization module 95 may use real-time EM tracking to generate a real-time location of the endoscope in a global coordinate system that may be registered to the patient's anatomy, represented by the preoperative model. In EM tracking, an EM sensor (or tracker) comprising of one or more sensor coils embedded in one or more locations and orientations in a medical instrument (e.g., an endoscopic tool) measures the variation in the EM field created by one or more static EM field generators positioned at a known location. The location information detected by the EM sensors is stored as EM data 93. The EM field generator (or transmitter), may be placed close to the patient to create a low intensity magnetic field that the embedded sensor may detect. The magnetic field induces small currents in the sensor coils of the EM sensor, which may be analyzed to determine the distance and angle between the EM sensor and the EM field generator. These distances and orientations may be intra-operatively "registered" to the patient anatomy (e.g., the preoperative model) in order to determine the geometric transformation that aligns a single location in the coordinate system with a position in the pre-operative model of the patient's anatomy. Once registered, an embedded EM tracker in one or more positions of the medical instrument (e.g., the distal tip of an endoscope) may provide real-time indications of the progression of the medical instrument through the patient's anatomy.

Robotic command and kinematics data 94 may also be used by the localization module 95 to provide localization data 96 for the robotic system. Device pitch and yaw resulting from articulation commands may be determined during pre-operative calibration. Intra-operatively, these calibration measurements may be used in combination with known insertion depth information to estimate the position of the instrument. Alternatively, these calculations may be analyzed in combination with EM, vision, and/or topological modeling to estimate the position of the medical instrument within the network.

As FIG. 15 shows, a number of other input data can be used by the localization module 95. For example, although not shown in FIG. 15, an instrument utilizing shape-sensing fiber can provide shape data that the localization module 95 can use to determine the location and shape of the instrument.

The localization module 95 may use the input data 91-94 in combination(s). In some cases, such a combination may use a probabilistic approach where the localization module 95 assigns a confidence weight to the location determined from each of the input data 91-94. Thus, where the EM data may not be reliable (as may be the case where there is EM interference) the confidence of the location determined by the EM data 93 can be decrease and the localization module 95 may rely more heavily on the vision data 92 and/or the robotic command and kinematics data 94.

As discussed above, the robotic systems discussed herein may be designed to incorporate a combination of one or more of the technologies above. The robotic system's computer-based control system, based in the tower, bed and/or cart, may store computer program instructions, for example, within a non-transitory computer-readable storage medium such as a persistent magnetic storage drive, solid state drive, or the like, that, upon execution, cause the system to receive and analyze sensor data and user commands, generate control signals throughout the system, and display the navigational and localization data, such as the position of the instrument within the global coordinate system, anatomical map, etc.

## 2. Multifunction End Effectors with Rotational Offsets.

This section describes multifunction end effectors with rotational offsets. In some embodiments, the multifunction end effectors described herein can be used with the robotically-enabled medical systems described above with reference to FIGs. 1-15. In other embodiments, the multifunction end effectors can be configured for manual use (i.e., non-robotic use). As will be described in greater detail below, the multifunction end effectors can be configured to perform multiple (e.g., two or more) functions. For example, a multifunction end effector can be configured to perform both cutting and grasping. Further, the multifunction end effectors can include a rotational offset that can be configured to preclude or hide one of the functions while the other function is performed so as to reduce the likelihood of or wholly prevent the accidental performance of the precluded or hidden function.

## A. Introduction to Multifunction End Effectors.

As mentioned briefly above, in some embodiments, robotically-enabled medical systems can include end effectors. As used in this section, the term "end effector" generally refers to a surgical or medical tool that is positioned at a distal end of a medical instrument and that is operable (e.g., remotely, manually, or robotically) to perform one or more functions during a medical procedure (e.g., laparoscopy or endoscopy). For example, an end effector can comprise a cutting tool (e.g., scissors), a gripping or grasping tool, needle drivers, etc.

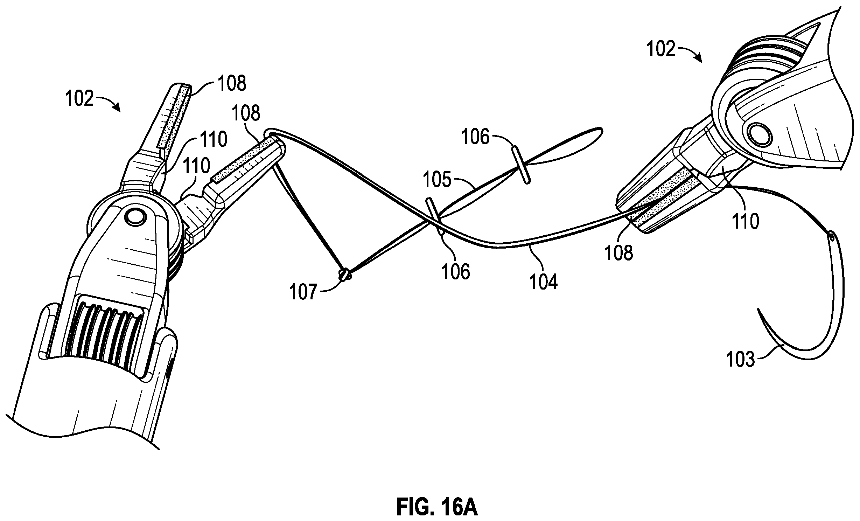

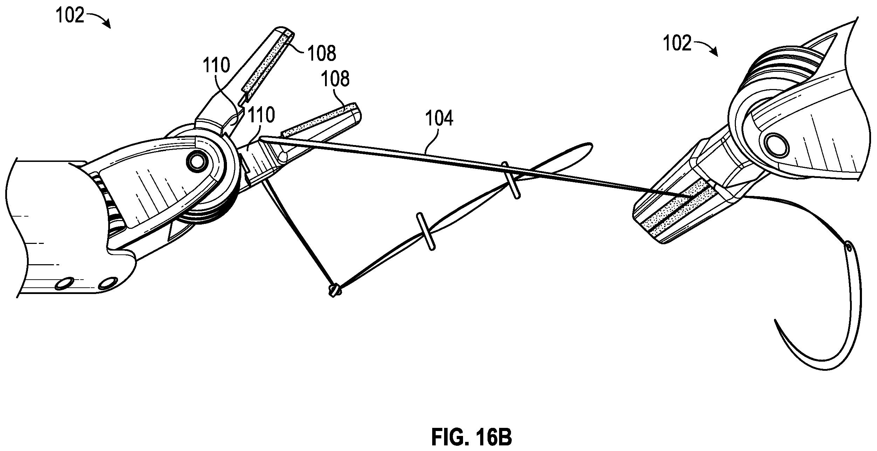

In some instances, multiple types of end effectors are needed during a medical procedure to accomplish a task. For example, suturing generally is performed using at least two end effectors to drive the needle through tissue, manipulate the suture, and break or cut the suture after a knot has been tied (see, for example, FIGs. 16A and 16B, described in greater detail below). Driving the needle and manipulating the suture generally requires at least two end effectors that are configured for grasping. In some instances, breaking the suture can be accomplished with a pair of grasping type end effectors, but that can be difficult. As such, many physicians desire an end effector that is configured for cutting (e.g., scissors) to cut the suture after a knot has been tied. Thus, during some procedures, a physician may desire or need to replace or exchange one type of end effector (e.g., a grasper) for another type of end effector (e.g., a cutting tool) during the procedure.

Replacing or exchanging end effectors during a medical procedure, however, may involve removing and replacing a medical instrument positioned endoscopically or laparoscopically within a patient's anatomy. This can be time consuming and difficult. Accordingly, minimizing end effector exchanges can be advantageous. For example, minimizing end effector exchanges can reduce total operation time, which can affect patient recovery time and operating room costs.

One method for minimizing tool exchanges is to use end effectors that are configured to perform multiple functions. These types of end effectors, which can perform multiple functions, are referred to in this application as multifunction end effectors, combined end effectors, or the like. One example of a multifunction end effector is an end effector that is configured for both cutting and grasping, although multifunction end effectors can be configured to perform various other combinations of functions. In some embodiments, the multifunction end effectors can perform two functions, while in other embodiments, the multifunction end effectors can perform three, four, or more functions.

FIG. 16A illustrates an embodiment of two multifunction end effectors 102 performing a suturing task. During the procedure, the two end effectors 102 manipulate a needle 103 and suture thread 104 to close an incision or laceration 105. The end effectors 102 manipulate the needle 103 and the suture thread 104 to execute one or more stitches 106 and form a knot 107. To accomplish this, in the illustrated embodiment, the multifunction end effectors 102 include a gripping portion 108. The gripping portion 108 is configured to grip or grasp the needle 103 and/or the suture thread 104, as shown in FIG. 16A. After the suture has been executed, the physician may desire to cut the excess suture thread 104. Accordingly, the multifunction end effectors 102 also include a cutting portion 110.

To use either the gripping portion 108 or the cutting portion 110, the physician must manipulate the end effector 102 such that the object to be acted on (e.g., the suture thread 104) is positioned within either the gripping portion 108 or the cutting portion 110 as desired.

One problem with some multifunction end effectors (such as the multifunction end effectors 102 illustrated in FIG. 16A) is that while one function may be desired, another function may be performed accidentally. For example, with respect to multifunction end effectors 102 having a both a gripping portion 108 and cutting portion 110, if the suture thread 104 slips or the multifunction end effectors 102 are not appropriately positioned, the suture thread 104 may be accidentally cut instead of gripped.

FIG. 16B illustrates one example of this problem using the multifunction end effectors 102 of FIG. 16A. In this example, the physician may desire to grasp the suture thread 104 with the gripping portion 108 of the left multifunction end effector 102. As shown, however, the suture thread 104 has slipped past the gripping portion 108 and now lies on the cutting portion 110. This can result in the suture thread 104 accidentally being cut. It will be appreciated that the suture thread 104 may be accidentally cut when the physician closes the jaws of the multifunction end effector 102 or merely by contact between the suture thread 104 and the cutting portion 110, even while the jaws remain open.

In some embodiments, the problem with some multifunctional end effectors 102 illustrated in FIG. 16B can occur because there is no differentiation between the two functions (gripping and cutting). For example, in the illustrated embodiment, the surface of the gripping portion 108 and the surface of the cutting portion 110 are substantially aligned, which can lead to accidental cutting when gripping.

## B. Example Multifunction End Effectors with Rotational Offsets.

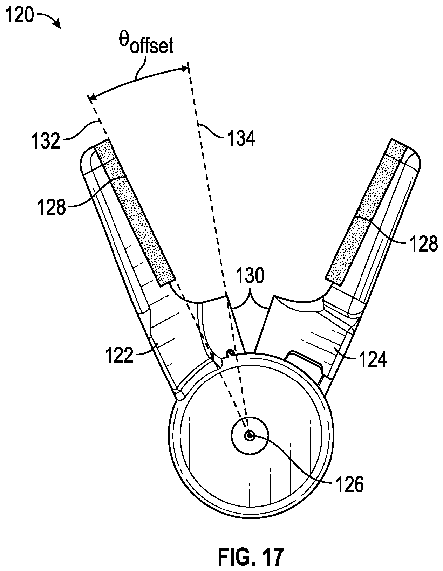

FIG. 17 illustrates an embodiment of a multifunction end effector 120 that includes a novel rotational offset $\Theta_{offset}$. As will be described below, in some embodiments, the rotational offset may be configured to reduce or prevent the problems discussed above with reference to the multifunction end effectors 102 of FIGs. 16A and 16B.

The multifunction end effector 120 includes a first body 122 and a second body 124. The first body 122 and the second body 124 can each be configured to rotate or pivot about the same or substantially the same axis 126 (sometimes referred to as the pivot axis). In some embodiments, a pivot axis can be substantially shared by the first body 122 and the second body 124, wherein the first body 122 and the second body 124 are configured for rotational movement about the pivot axis. In some embodiments, a single pivot pin extends along the pivot axis 126 and through each of the first body 122 and the second body 124. In other embodiments, a pair of aligned or substantially aligned pivot pins extends along the pivot axis 126, wherein one of the pivot pins extends through the first body 122 and the other extends through the second body 124. In the illustrated orientation, the axis 126 extends into and out of the page and permits rotation of the first body 122 and the second body 124 about the axis 126 in the plane of the page. Although not illustrated, the first body 122 and the second body 124 can each be connected to one or more cables or pull wires that are actuable to control the rotation of the first body 122 and the second body 124. Thus, the multifunction end effector 120 can be controlled by actuation of the pull wires. In some embodiments, the first body 122 and the second body 124 are each independently controllable by their own individual cables or pull wires. Other methods for actuating the rotation of the first body 122 and the second body 124, such as using motors, is also possible. In some embodiments, the first body 122 and the second body 124 can be configured rotate about different axes, such that the axis of rotation of the first body 122 is not the same as the axis of rotation of the second body 124.

Each of the first body 122 and the second body 124 can comprise an elongated portion that extends from a spherical or circular portion. The elongated portion can be configured to perform one or more functions (e.g., gripping or cutting). The circular portion can comprise an opening for receiving one or more pivot pins therein, thereby allowing the first body 122 and the second body 124 to pivot around the pivot axis. Other shapes are also possible.

The multifunction end effector 120 is configured to perform multiple (e.g. two or more) functions. In some embodiments, each of the first body 122 and the second body 124 can include more than one functional portion. In the illustrated embodiment, each of the first body 122 and the second 124 includes a first functional portion (gripping portion 128) and a second functional portion (cutting portion 130). In the illustrated embodiment, the gripping portion 128 is positioned distally of the cutting portion 130, although this may be reversed in some embodiments. Further, while the multifunction end effector 120 has been illustrated with a gripping portion 128 and a cutting portion 130 to perform gripping and cutting, respectively, in other embodiments, the multifunction end effector 120 can be configured to perform other functions besides gripping and cutting.

In some embodiments, the end effector 120 can be configured with multiple portions for performing multiple end effector functions (e.g., two, three, four, or more functions). The multiple portions for performing multiple end effector functions can be combined so that they can be controlled about a single axis (e.g., the pivot axis 126). In some embodiments, the multiple portions for performing multiple end effector functions can be combined so that they can be controlled via a single degree of freedom. In the illustrated example, the single degree of freedom may be rotation about the pivot axis 126.

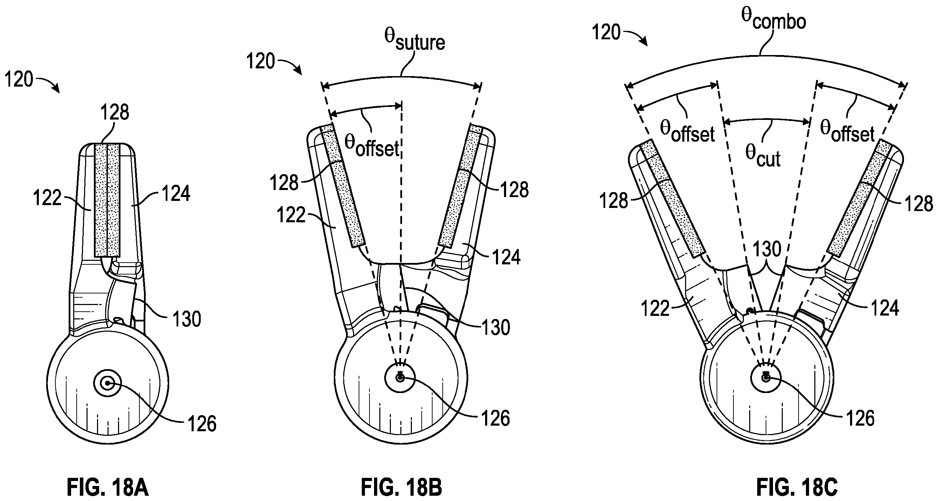

As shown in FIG. 17, the multifunction end effector 120 includes a rotational offset $\Theta_{offset}$ between the gripping portion 128 and the cutting portion 130 on each of the first body 122 and the second body 124. In some embodiments, the rotational offset $\Theta_{offset}$ is measured as the angle between a first reference line (e.g., a gripping portion reference line) 132 and a second reference line (e.g., a cutting portion reference line) 134. The gripping portion reference line 132 can be defined as a line extending between the pivot axis 126 and the distal tip of the gripping portion 128. In the illustrated embodiment, a face of the gripping portion 128 lies on the gripping portion reference line 132. In other embodiments, a face of the gripping portion 128 need not co-align with the gripping portion reference line 132. The illustrated configuration can allow the face of the gripping portion 128 of the first body 122 to contact and press against the face of the gripping portion 128 of the second body 124 when the first body 122 and the second body 124 are rotated to a closed position (for example, as shown in FIG. 18A). The cutting portion reference line 134 can be defined as a line extending between the pivot axis 126 and the distal tip of the cutting portion 130. In the illustrated embodiment, a face of the cutting portion 130 does not lie on the cutting portion reference line 134. Instead, in this embodiments, the face is angled slightly with respect to the cutting portion reference line 134 as shown. In other embodiments, a face of the cutting portion 130 can co-align with the cutting portion reference line 134. The illustrated configuration can allow a face of the cutting portion 130 of the first body 122 to shear past a face of the cutting portion 130 of the second body 124 or a rotational range as the first and second bodies 122, 124 are rotated. In some embodiments, the cutting portion 130 can also be angled past a center line to act similar to hooked scissors.

In some embodiments, inclusion of rotational offset $\Theta_{offset}$ between the different functional portions (e.g., the gripping portion 128 and the cutting portion 130) can be configured to allow the multifunction end effector 120 to operate in various modes. In some embodiments, a mode may permit access to one functional portion while precluding or hiding access to another functional portion. For example, in a first embodiment, with respect to multifunction end effector 120 illustrated in FIG. 17, the end effector 120 can have a first mode, (e.g., a "suture mode") in which only the gripping portion 128 is capable of utilization, and a second mode (e.g., a "cut mode"), in which only the cutting portion 130 is available for use. These modes may be advantageous because they can prevent inadvertent performance of an undesired function. For example, if a physician desires grasping, the multifunction end effector can be used in suture mode, which permits grasping and precludes cutting.

In other embodiments, the end effector 120 can have a first mode (e.g., a "suture mode") in which only the gripping portion 128 is capable of utilization and a second mode (e.g., a "combination mode" or "all-encompassing mode") in which both the gripping portion 128 and cutting portion 130 are available. In other words, the first mode may be encompassed by the second mode in the combination mode. The suture mode may be advantageous because it can prevent inadvertent cutting. For example, if a physician desires grasping, the multifunction end effector can be used in suture mode, which permits grasping and precludes cutting. The combination mode may be beneficial in some instances as it may provide more freedom to a physician when desired because it allows access to both functions of the multifunction end effector.

In some embodiments, certain modes advantageously exclude one or more functions of the multifunction end effector.

As will be discussed in more detail below, in some embodiments, the different modes can be controlled via software or by mechanical components. For example, software executed on robotically-enabled medical system may limit rotation of the first and second bodies 122, 124 such that the multifunction end effector 120 remains in a certain mode. As another example, the multifunction end effector 120 could include a hard stop on the first and second bodies 122, 124 that could be engaged or disengaged to control mode selection.

FIGs. 18A-18C illustrate the multifunction end effector 120 of FIG. 17 in various positions. FIG. 18A illustrates the multifunction end effector 120 in a closed position. FIG. 18B illustrates the multifunction end effector 120 in a first open position. FIG. 18C illustrates the multifunction end effector 120 in a second open position. As shown in FIGs. 18A-18C, to move between the closed position, the first open position, and the second position, the bodies 122, 124 are rotated or pivoted relative to each other around the pivot axis 126.

In the closed position (FIG. 18A), the first body 122 and second body 124 are rotated together such that the gripping portions 128 contact each other. During use, the first body 122 and second body 124 can be rotated together such that the gripping portions 128 contact an item positioned there between, such as suture thread or a needle, for example.

In the illustrated first open position (FIG. 18B), the first body 122 and the second body 124. are rotated apart so as to expose the faces of the gripping portions 128. Notably, because of the rotational offset $\Theta_{offset}$, while the gripping portions 128 are exposed, the cutting portions 130 still overlap and thus are not exposed or accessible. Thus, in some embodiments, it is possible to operate the multifunction end effector 120 in a mode that permits access to the gripping portions 128 while precluding access to the cutting portions 130 by limiting relative rotation of the first and second bodies 122, 124 to a rotational range from the closed position (FIG. 18A) to the first open position of FIG. 18B illustrated as $\Theta_{suture}$. This can be considered the suture mode discussed above. In some embodiments, in the suture mode, the cutting portions 130 of each of the bodies 122, 124 overlap, such that there is no opening between the cutting portions 130. This overlap creates a mechanical "safety lock" or stop, whereby a suture would not be able to be accidentally cut. In other words, during the suture mode, there is advantageously minimal to zero risk that a suture may be accidentally cut by the cutting portions 130.

In the illustrated second open position (FIG. 18C), the first body 122 and the second body 124 are rotated further apart (as compared with the first open position of FIG. 18B) so as to expose the faces of the gripping portions 128 and the cutting portions 130. When the first and second bodies 122, 124 are rotated past $\Theta_{suture}$ the cutting portions 130 become available. In some embodiments, the multifunction end effector can be operated in a cut mode or a combination mode as discussed above. In some embodiments, in cut mode, rotation of the first and second bodies 122, 124 is limited to the range of angles between the first open position (FIG. 18B) and the angle $\Theta_{cut}$. This mode allows access and operation of the cutting portions 130 but precludes access and operation of the gripping portions 128. In some embodiments, in the combination mode, the multifunction end effector 120 can operate in a range of angles from the closed position (FIG. 18A) to the angle illustrated as $\Theta_{combo}$ in FIG. 18C. This can allow access to both the gripping portions 128 and the cutting portions 130.

As illustrated in the examples of FIGs. 18A-18C, the rotational offset $\Theta_{offset}$ can be configured to allow ranges of angles in which certain functionality is accessible and certain functionality is precluded or hidden. As will be discussed further below, in certain embodiments, rotation of the bodies 122, 124 of the multifunction end effector 120 can be limited to certain of these ranges to permit operation of the multifunction end effector 120 in certain modes.

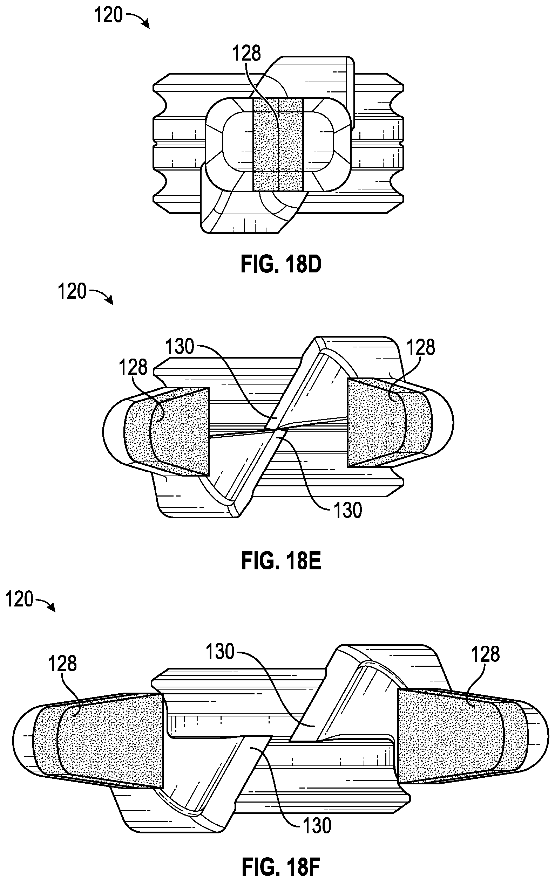

FIGs. 18D-18F are top views of the multifunction end effector 120 corresponding to the closed position of FIG. 18A, the first open position of FIG. 18B, and the second open position of FIG. 18C, respectively. As shown in FIG. 18D, in the closed configuration, the gripping portions 128 contact each other. As shown in FIG. 18E, in the first open configuration (representative of the suture mode, for example), the bodies 122, 124 have been rotated open such that the gripping portions 128 are spaced apart; however, rotation of the bodies 122, 124 can be limited such that the cutting portions 130 still overlap and thus do not form any space or angle between them. In this position, a suture or other object is wholly or substantially prevented from inadvertently being accessed and cut by the cutting portions. FIG. 18E also illustrates that the cutting portions 130 can be formed at an angle (e.g., in the shape of blade) that terminates at a sharp cutting point. FIG. 18F illustrates a top view of the multifunction end effector 120 in the second open configuration (representative of the cut mode or combo mode, for example). As shown, the bodies 122, 124 have been further rotated so as to create a gap, space, or angle between the cutting portions 130. In this configuration, the cutting portions 130 are accessible. That is, the multifunction end effector 120 can be moved such that a suture or other object is positioned in the gap between the cutting portions 130 and then the bodies 122, 124 can be rotated together to cut the suture.

In some embodiments, in suture mode the rotation of the bodies 122, 124 is limited between the position shown in FIGs. 18A and 18D (the closed configuration) and the position shown in FIGs. 18B and 18E (the first open configuration), That is, the bodies 122, 124 can be rotated between the closed configuration to a configuration that creates a space between the gripping portions 128 but that maintains an overlap between the cutting portions 130 such that access to the cutting portions is precluded.

In some embodiments, in cut mode the rotation of the bodies 122, 124 is limited between the position shown in FIGs. 18B and 18E (the first open configuration) and the position shown in FIGs. 18C and 18F (the second open configuration), That is, the bodies 122, 124 can be rotated between the second open configuration which allows access to the cutting portions 130 by creating a space or gap there between and the first open configuration that closes the gap between the cutting portions 130 to execute a cut function. At the same time, in the cut mode, the bodies 122, 124 can be limited from further rotation such that the gripping portions 128 cannot be brought together.

In some embodiments, in combination mode the rotation of the bodies 122, 124 is limited between the position shown in FIGs. 18A and 18D (the closed configuration) and the position shown in FIGs. 18C and 18F (the second open configuration). In this mode, bodies can be rotated to angles that permit access and use of both the cutting and gripping functions.

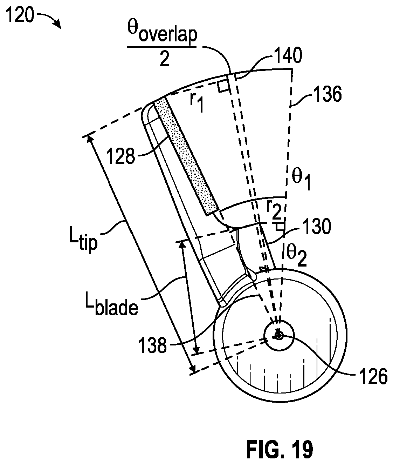

In some embodiments, the rotational offset $\Theta_{offset}$ can be defined with relation to the various features shown in FIG. 19A. These features are provided by way of illustrative example and are not intended to be limiting. As shown in FIG. 19A, a center line 136 can be a bisecting line between the two bodies 122, 124 of the multifunction end effector 120 when the bodies 122, 124 are set open to their widest position, referred to as $\theta_{max,open}$. Gripping portion and cutting portion reference lines 138, 140 can be formed between the pivot axis 126 and distal tips of the gripping portion 128 and the cutting portion 130. In the illustrated embodiment, the end effector reference lines 138, 140 have lengths $L_{tip}$ (for the gripping portion 128) and $L_{blade}$ (for the cutting portion 130) as shown. An angle $\theta_1$ can describe the angle formed between the center line 136 and the gripping portion reference line 138. An angle $\theta_2$ can describe the angle formed between the center line 136 and the cutting portion reference line 140.

In some embodiments, the following relationships can be established:

$$\theta_{max,open} = 2\theta_1 \qquad\qquad \text{(Eq. 1)}$$

$$\theta_{2,open} = 2\theta_2 \qquad\qquad \text{(Eq. 2)}$$

$$\theta_{1,open} = 2\left(\theta_1 - \left(\theta_2 + \frac{\theta_{overlap}}{2}\right)\right) = 2\theta_{offset} \qquad\qquad \text{(Eq. 3)}$$

In some embodiments, $\theta_{overlap}$ (which is a buffer region within the gripping region, adjacent to the cutting region) may be nonzero so that there is a rotational safety margin between the gripping function and the cutting function. In some embodiments, the $\theta_{overlap}$ is at least 1, 2.5, 5 degrees or higher.

In some embodiments, the max open angle $\theta_{max,open}$ determines the largest object that can be grasped between the tips of gripping portions 128. At the distal tip of the gripping portions 128, at $\theta_{max,open}$ the distance between the gripping portions 128 is equal to twice the radius $r_1$. Equation 4 establishes the relationship between the radius $r_1$ and the rotational offset $\Theta_{offset}$:

$$2r_1 = 2(L_{tip} \sin\theta_{offset}) > \text{Suture diameter} \approx 1 \text{ mm} \qquad \text{(Eq. 4)}$$

Therefore:

$$\theta_{offset} > \sin^{-1}\left(\frac{r_1}{L_{tip}}\right) \qquad\qquad \text{(Eq. 5)}$$

In some embodiments, the distance between the distal tips of the cutting portions 130 is equal to twice the illustrated radius $r_2$. Equation 6 establishes the relationship between the radius $r_2$ and the rotational offset $\Theta_{offset}$:

$$2r_2 = 2(L_{blade} \sin\theta_2) > \text{Suture diameter} \approx 1 \text{ mm} \qquad \text{(Eq. 6)}$$

In some embodiments, $\theta_{1,open}$ is at least 30 degrees for end effectors configured for medical procedures as described above. Accordingly, a minimum value of the rotational offset $\theta_{offset}$ can be determined from Equation 7.

$$\theta_{1,open} \geq 30^{\circ} = 2\left(\theta_1 - \left(\theta_2 + \frac{\theta_{overlap}}{2}\right)\right) = 2\theta_{offset} \qquad \text{(Eq. 7)}$$

Consequently, in this example, the rotational offset $\Theta_{offset}$ should be greater than or equal to 15 degrees. 15 degrees is merely one example or the rotational offset $\Theta_{offset}$. It will readily be appreciated that other values for the rotational offset $\Theta_{offset}$ could also be used. For example, in some embodiments, the rotational offset $\Theta_{offset}$ is at least 5 degrees, at least 10, degrees, at least, 15 degrees, or at least 20 degrees.

In some embodiments, rotationally offsetting a feature, such as a cutting portion, from a second feature, such as a gripping portion can create distinct ranges, or modes. Advantageously, the different modes can be controlled about a single pivot by expanding the gripping portions about different angular ranges. These ranges or modes can, in some embodiments, be further implemented or reinforced in software. For example, in the multifunction end effector of FIGs. 16A-19, the cutting portion 130 is not exposed until the gripping portion angle opens to at least twice the rotational offset $\Theta_{offset}$. Limiting the open angle in software, such that access to the second range of angles can only be accessed through user input, would prevent inadvertent suture cuts. In some embodiments, surgeons could alternate between the two modes by hand motions, such as clicking the master hand grips twice or touching a button on the master controller.

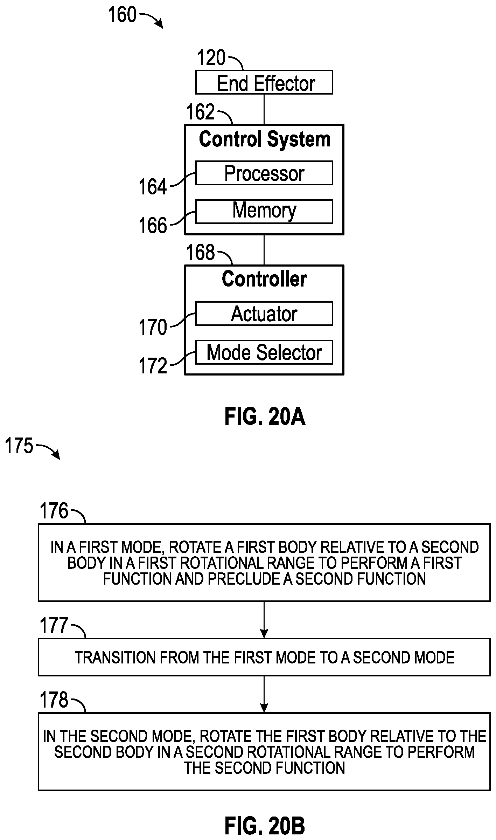

FIG. 20 illustrates a block diagram of system 160. The system can be a robotically-enabled medical system as discussed above with reference to FIGs. 1-15. The system 160 includes the multifunction end effector 120. The multifunction end effector 120 can be positioned on a distal tip of a medical instrument. The medical instrument can be manipulated by a robotic arm.

In this example, the end effector 120 is connected to a control system 162. According to the invention, the control system 162 includes a processor 164 and a memory 166. The memory 166 includes instructions that, when executed by the processor 164, cause the control system 162 to control the end effector 120. The instructions stored in the memory 166 cause the processor 164 to limit rotation of the end effector 120 to certain ranges, such that the end effector 120 is operable in a plurality of modes (e.g., the suture mode, cut mode, and combination mode discussed above).

The system 160 can also include a controller 168. A physician may provide inputs on the controller 168 that can be provided to the processor 164 to control the end effector 120. In the illustrated embodiment, the controller 168 includes an actuator 170 and a mode selector 172. The actuator 170 may be used to, among other things, control rotation of the bodies of the end effector 120 (i.e., to either open or close the end effector 120). The mode selector 172 can be configured to allow the physician to select between the available modes (e.g., the suture mode, cut mode, and combo mode discussed above).

When a mode is selected that precludes certain functionality of the end effector 120 the rotation of the end effector 120 can be appropriately limited. For example, if the mode selector 172 is used to select suture mode, the system 160 can limit rotation of the end effector 120 such that even if the physician uses the actuator 170 to open the end effector 120 as wide as possible, the cutting portions of the end effector 120 will remain hidden. That is, the end effector 120 will only rotate open to a point where access to the cutting portions is still precluded.

FIG. 20B is a flowchart illustrating an example method 175 for implementing a multifunction end effector having multiple operating modes. The method 175 can be implemented, for example, by the system 160 of FIG. 20A. The method 175 begins at block 176. At block 176, the end effector is operated in a first mode. In the first mode, the method 175 includes rotating a first body relative to a second body in a first rotational range to perform a first function and preclude a second function. According to the invention, the first function is gripping and the second function is cutting. In some embodiments, rotation is limited to preclude access to the second function by using a rotational offset as described above.

The method 175 can proceed to block 177 at which the end effector is transitioned from the first mode to a second mode. In some embodiments, this can involve permitting via software, the end effector to operate in a different rotation range. In some embodiments, this can involve removing or disengaging physical hard stops on the end effector.

Next, at block 178, the end effector is operated in the second mode. In some embodiments, this includes rotating the first body relative to the second body in a second rotational range that allows access to the second function. According to the invention, the second rotational range precludes access to the first function.

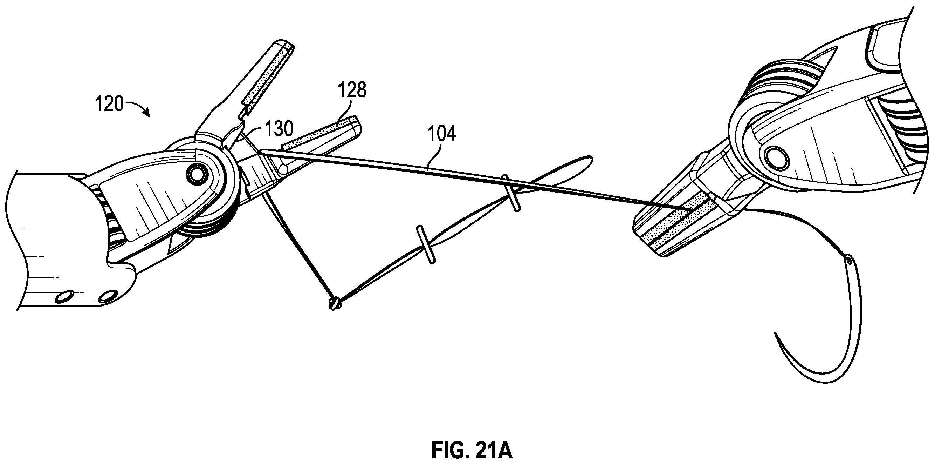

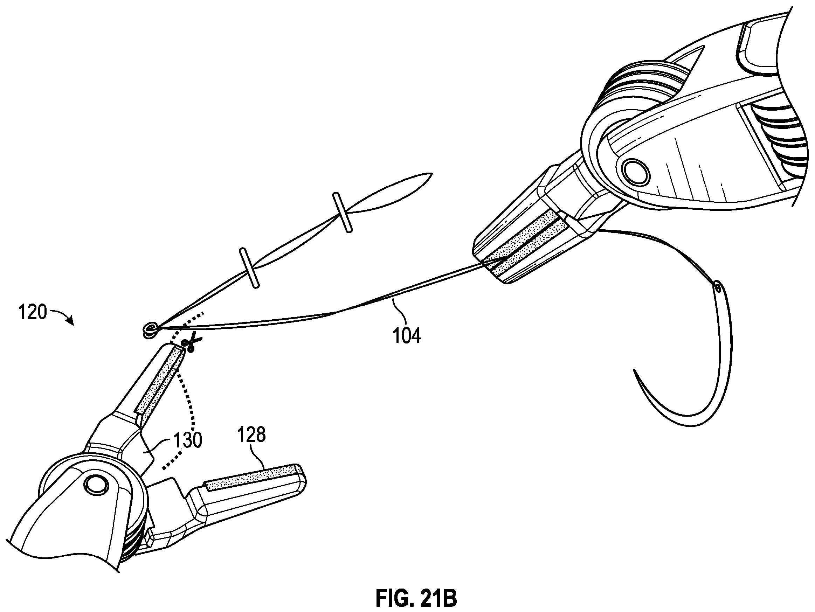

FIGs. 21A and 21B illustrate an example suturing procedure with the multifunctional end effector 120. In FIG. 21A, the end effectors 120 are shown in suture mode. As such rotation of the end effectors 120 is limited to preclude access to the cutting portions 130. In this configuration, the gripping portions 128 can be used to manipulate the suture thread 104. As shown, even if the suture thread 104 slips down the face of the gripping portions 128, it cannot enter the cutting portions 130 because of the rotational offset. As such, inadvertent cutting of the suture thread can be prevented. For example, compare FIG. 21A (which shows an end effector 120 with a rotation offset) with FIG. 16B (which shows an end effector 102 without a rotational offset).

In FIG. 21B, the end effector 120 has been set to either cut mode or combination mode such that the cutting portions 130 are accessible. In this mode, the end effector 120 can be used to cut the suture thread 104.

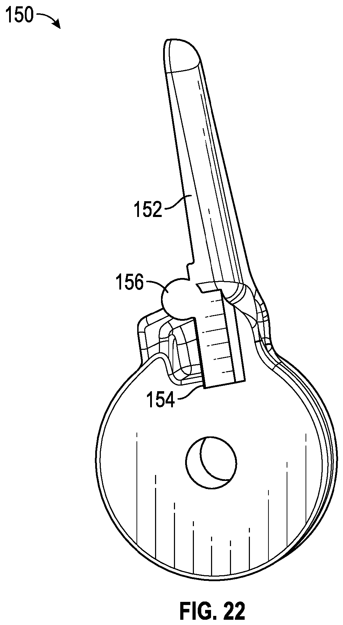

FIG. 22 illustrates a body 150 for a multifunction end effector that uses a protrusion 156 to separate various functional portions instead of a rotational offset. In some embodiments, the body 150 for a multifunction end effector can include the protrusion 156 that longitudinally separates a gripping portion 152 from a cutting portion 154, so that the edge of the cutting portion 154 is generally still close to in line with the face of the gripping portion 152. The protrusion 156 would protrude out, however, and may still require the same increased rotation for the suture to gain access to the cutting portion 154. In this embodiment, rotation of the body 150 can be limited such that the protrusion 156 blocks access to the cutting portion 154. To access the cutting portion 154, the body 150 can be rotated wider so as to allow an object to pass the protrusion 156 so as to be accessed by the cutting portion 154.

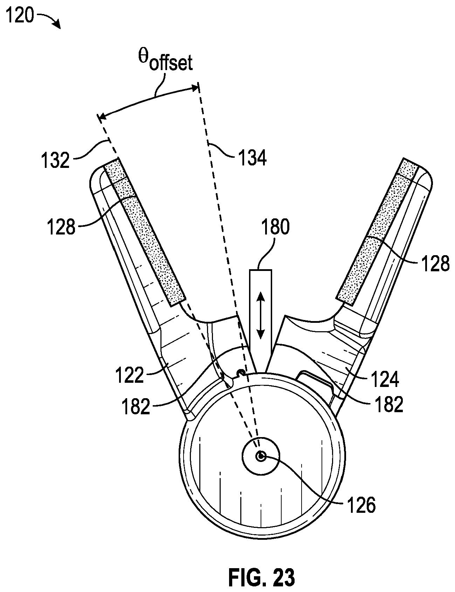

While the embodiments described above include a multifunctional end effector 120 having a first body 122 and a second body 124 with more than one active functional portion, in other embodiments, the multifunction end effector 120 can comprise a first body 122 and a second body 124 each with a gripping portion and an offset blocking surface 182, for example as shown in FIG. 23. In this example, the first body 122 and the second body 124 can comprise similar bodies as shown in FIG. 17 without the cutting edge of the cutting portion 130. Rather, these portions simply serve as blocking surfaces 182 that prevent exposure of an internal component 180 (e.g., a push blade, an ablation or heating component, a laser) until the first body 122 and second body 124 are opened wide enough. In some embodiments, once the blocking surfaces 182 are rotated apart, the internal component 180 can be pushed up between the blocking surfaces 182 in the direction indicated by the arrow in FIG. 23. After use, the internal component 180 can then be retracted. In other words, the multifunctional end effector 120 would still have a first mode for gripping and a second mode for cutting, ablation, etc. that is blocked or excluded from the first mode.

While the embodiments described above include a multifunctional end effector 120 having a first body 122 and a second body 124 each with a rotationally offset portion, in other embodiments, only one of the first body 122 and the second body 124 comprises a rotationally offset portion. The rotationally offset portion of the single body (e.g., first body 122 or second body 124) can be used during a first mode (e.g., suturing or gripping) to preclude operation of the end effector 120 in a second mode (e.g., cutting). Furthermore, while the embodiments described above discuss a multifunctional end effector 120 in the form of a combined grasper and cutter, other combinations are also possible. For example, in some embodiments, the multifunctional end effector 120 can comprise a combined hooked scissors and straight/curved scissors, wherein the straight/curved scissors can be hidden. The hooked scissors can allow complete enclosure of vessels before cutting, while the straight/curved scissors can allow cutting of objects smaller than the vessel diameter. In some embodiments, the multifunctional end effector 120 can comprise a combined scissors and monopolar hook/spatula, wherein the monopolar hook/spatula can be hidden. In some embodiments, the multifunctional end effector 120 can comprise a combined gripper/grasper and monopolar hook/spatula, wherein the monopolar hook/spatula can be hidden. In some embodiments, the multifunctional end effector 120 can comprise a combined pick/tenaculum and scissors, wherein the scissors can be hidden. In some embodiments, the multifunctional end effector 120 can comprise a combined bipolar grasper and scissors, wherein the scissors is hidden. In some embodiments, the multifunctional end effector 120 can comprise a speculum (e.g., instrument for dilating an orifice) and scissors, wherein the scissors is hidden. In some embodiments, the multifunctional end effector 120 can comprise wipers and a camera, wherein the camera is hidden.

### 3. Implementing Systems and Terminology.

Implementations disclosed herein provide systems, methods and apparatus for robotically-enabled medical systems. Various implementations described herein include multifunction end effectors with rotational offsets.

It should be noted that the terms "couple," "coupling," "coupled" or other variations of the word couple as used herein may indicate either an indirect connection or a direct connection. For example, if a first component is "coupled" to a second component, the first component may be either indirectly connected to the second component via another component or directly connected to the second component.

The position estimation and robotic motion actuation functions described herein may be stored as one or more instructions on a processor-readable or computer-readable medium. The term "computer-readable medium" refers to any available medium that can be accessed by a computer or processor. By way of example, and not limitation, such a medium may comprise random access memory (RAM), read-only memory (ROM), electrically erasable programmable read-only memory (EEPROM), flash memory, compact disc read-only memory (CD-ROM) or other optical disk storage, magnetic disk storage or other magnetic storage devices, or any other medium that can be used to store desired program code in the form of instructions or data structures and that can be accessed by a computer. It should be noted that a computer-readable medium may be tangible and non-transitory. As used herein, the term "code" may refer to software, instructions, code or data that is/are executable by a computing device or processor.

The methods disclosed herein comprise one or more steps or actions for achieving the described method. Unless specific order of steps or actions is required for proper operation of the method that is being described, the order and/or use of specific steps and/or actions may be modified.

As used herein, the term "plurality" denotes two or more. For example, a plurality of components indicates two or more components. The term "determining" encompasses a wide variety of actions and, therefore, "determining" can include calculating, computing, processing, deriving, investigating, looking up (e.g., looking up in a table, a database or another data structure), ascertaining and the like. Also, "determining" can include receiving (e.g., receiving information), accessing (e.g., accessing data in a memory) and the like. Also, "determining" can include resolving, selecting, choosing, establishing and the like.

The phrase "based on" does not mean "based only on," unless expressly specified otherwise. In other words, the phrase "based on" describes both "based only on" and "based at least on."

As used herein, the term "approximately" or "about" refers to a range of measurements of a length, thickness, a quantity, time period, or other measurable value. Such range of measurements encompasses variations of +/-10% or less, preferably +/-5% or less, more preferably +/-1% or less, and still more preferably +/-0.1% or less, of and from the specified value, in so far as such variations are appropriate in order to function in the disclosed devices, systems, and techniques.

The previous description of the disclosed implementations is provided to enable any person skilled in the art to make or use the present invention. Various modifications to these implementations will be readily apparent to those skilled in the art, and the generic principles defined herein may be applied to other implementations without departing from the scope of the invention. For example, it will be appreciated that one of ordinary skill in the art will be able to employ a number corresponding alternative and equivalent structural details, such as equivalent ways of fastening, mounting, coupling, or engaging tool components, equivalent mechanisms for producing particular actuation motions, and equivalent mechanisms for delivering electrical energy.

### Claims

1.   A medical system (160) comprising:

   a medical instrument including an end effector (120) comprising:

      a first body (122) that shares a pivot axis with a second body (124), the first body rotatable relative to the second body to permit a first mode of operation comprising a gripping mode and a second mode of operation comprising a cutting mode,
wherein during the first mode of operation the second mode of operation is inoperable, and wherein the first mode of operation and second mode of operation can be controlled substantially about one axis;

a non-transitory computer readable medium (166) having stored thereon executable instructions; and
a processor (164) in communication with the non-transitory computer readable medium and configured to execute the instructions to cause the system to at least:

operate the end effector in the first mode of operation in which the first body is rotatable relative to the second body in a first rotational range to perform a first function and preclude a second function;
transition the end effector from the first mode of operation to the second mode of operation; and
operate the end effector in the second mode of operation in which the first body is rotatable relative to the second body in a second rotational range different from the first rotational range to perform a second function and preclude the first function.

2. The medical system of Claim 1, wherein the first body includes a first engagement surface having a first portion (128) rotationally offset about the pivot axis from a second portion (130), and wherein the second body includes a second engagement surface having a first portion (128) rotationally offset about the pivot axis from a second portion (130).

3. The medical system of Claim 2, wherein the processor is further configured to execute the instructions to cause the system to at least:

operate the end effector in the first mode of operation to permit cooperation between the first portion of the first engagement surface and the first portion of the second engagement surface and preclude cooperation between the second portion of the first engagement surface and the second portion of the second engagement surface, and
transition the end effector from the first mode of operation to the second mode of operation to permit cooperation between at least the second portion of the first engagement surface and the second portion of the second engagement surface.

4. The medical system of Claim 2, wherein the first portion of the first body is rotationally offset from the second portion of the first body by at least 10 degrees.

5. The medical system of Claim 1, wherein the first body includes a first portion (128) and a second portion (130), wherein the first portion comprises a gripping portion and the second portion comprises a cutting portion.

6. The medical system of Claim 5, wherein during the first mode of operation, the cutting portion is unexposed.

7. The medical system of Claim 1, wherein both the first body and the second body comprise a gripping portion (128) and a cutting portion (130), and wherein during the first mode of operation, the cutting portions overlap.

8. The medical system of Claim 7, wherein during the second mode of operation, a recess is formed between the cutting portions.

9. The medical system of Claim 1, wherein in the first mode of operation, the end effector comprises a mechanical stop that makes the second mode of operation inoperable.

10. The medical system of Claim 9, wherein the mechanical stop comprises a hard stop formed on the first body and the second body of the end effector.

11. The medical system of Claim 1, further comprising a robotic arm coupled to the medical instrument.

12. The medical system of Claim 1, wherein the processor is further configured to execute the instructions to cause the system to transition the end effector from the first mode of operation or the second mode of operation to a third mode of operation in which the first body is rotatable relative to the second body in the first rotational range and the second rotational range to perform the first function and the second function.

**Patentansprüche**

1. Medizinisches System (160), umfassend:
   ein medizinisches Instrument, das einen Endeffektor (120) einschließt, umfassend:

   einen ersten Körper (122), der eine Schwenkachse mit einem zweiten Körper (124) teilt, wobei der erste Körper relativ zu dem zweiten Körper drehbar ist, um einen ersten Betriebsmodus, umfassend einen Greifmodus, und einen zweiten Betriebsmodus, umfassend einen Schneidmodus, zu ermöglichen,
   wobei während des ersten Betriebsmodus der zweite Betriebsmodus nicht betriebsbereit ist und wobei der erste Betriebsmodus und der zweite Betriebsmodus im Wesentlichen um eine Achse herum gesteuert werden können;
   ein nicht flüchtiges computerlesbares Medium (166), das ausführbare Anweisungen darauf gespeichert aufweist; und
   einen Prozessor (164), der mit dem nicht flüchtigen computerlesbaren Medium in Kommunikation steht und konfiguriert ist, um die Anweisungen auszuführen, um das System mindestens zu veranlassen zum:

   Betreiben des Endeffektors in dem ersten Betriebsmodus, in dem der erste Körper relativ zu dem zweiten Körper in einem ersten Drehbereich drehbar ist, um eine erste Funktion durchzuführen und eine zweite Funktion auszuschließen;
   Übergehen des Endeffektors von dem ersten Betriebsmodus in den zweiten Betriebsmodus; und
   Betreiben des Endeffektors in dem zweiten Betriebsmodus, in dem der erste Körper relativ zu dem zweiten Körper in einem zweiten Drehbereich drehbar ist, der sich von dem ersten Drehbereich unterscheidet, um eine zweite Funktion durchzuführen und die erste Funktion auszuschließen.

2. Medizinisches System nach Anspruch 1, wobei der erste Körper eine erste Eingriffsoberfläche einschließt, die einen ersten Abschnitt (128) aufweist, der um die Schwenkachse von einem zweiten Abschnitt (130) drehend versetzt ist, und wobei der zweite Körper eine zweite Eingriffsoberfläche einschließt, die einen ersten Abschnitt (128) aufweist, der um die Schwenkachse herum von einem zweiten Abschnitt (130) drehend versetzt ist.

3. Medizinisches System nach Anspruch 2, wobei der Prozessor ferner konfiguriert ist, um die Anweisungen auszuführen, um das System mindestens zu veranlassen zum:

   Betreiben des Endeffektors in dem ersten Betriebsmodus, um ein Zusammenwirken zwischen dem ersten Abschnitt der ersten Eingriffsoberfläche und dem ersten Abschnitt der zweiten Eingriffsoberfläche zu ermöglichen und das Zusammenwirken zwischen dem zweiten Abschnitt der ersten Eingriffsoberfläche und dem zweiten Abschnitt der zweiten Eingriffsoberfläche zu verhindern, und
   Überführen des Endeffektors von dem ersten Betriebsmodus in den zweiten Betriebsmodus, um das Zusammenwirken zwischen mindestens dem zweiten Abschnitt der ersten Eingriffsoberfläche und dem zweiten Abschnitt der zweiten Eingriffsoberfläche zu ermöglichen.

4. Medizinisches System nach Anspruch 2, wobei der erste Abschnitt des ersten Körpers von dem zweiten Abschnitt des ersten Körpers um mindestens 10 Grad drehend versetzt ist.

5. Medizinisches System nach Anspruch 1, wobei der erste Körper einen ersten Abschnitt (128) und einen zweiten Abschnitt (130) einschließt, wobei der erste Abschnitt einen Greifabschnitt umfasst und der zweite Abschnitt einen Schneidabschnitt umfasst.

6. Medizinisches System nach Anspruch 5, wobei während des ersten Betriebsmodus der Schneidabschnitt nicht freiliegt.

7. Medizinisches System nach Anspruch 1, wobei sowohl der erste Körper als auch der zweite Körper einen Greifabschnitt (128) und einen Schneidabschnitt (130) umfassen und wobei sich die Schneidabschnitte während des ersten Betriebsmodus überlappen.

8. Medizinisches System nach Anspruch 7, wobei während des zweiten Betriebsmodus eine Aussparung zwischen den Schneidabschnitten ausgebildet wird.

9. Medizinisches System nach Anspruch 1, wobei der Endeffektor in dem ersten Betriebsmodus einen mechanischen Anschlag umfasst, der den zweiten Betriebsmodus in einen nicht betriebsbereiten Zustand versetzt.

10. Medizinisches System nach Anspruch 9, wobei der mechanische Anschlag einen harten Anschlag umfasst, der auf dem ersten Körper und dem zweiten Körper des Endeffektors ausgebildet ist.

11. Medizinisches System nach Anspruch 1, ferner umfassend einen Roboterarm, der mit dem medizinischen Instrument gekoppelt ist.

12. Medizinisches System nach Anspruch 1, wobei der Prozessor ferner konfiguriert ist, um die Anweisungen auszuführen, um das System zu veranlassen, den Endeffektor von dem ersten Betriebsmodus oder dem zweiten Betriebsmodus in einen dritten Betriebsmodus zu überführen, in dem der erste Körper relativ zu dem zweiten Körper in dem ersten Drehbereich und dem zweiten Drehbereich drehbar ist, um die erste Funktion und die zweite Funktion durchzuführen.

**Revendications**

1. Système médical (160), comprenant :
   un instrument médical comportant un effecteur terminal (120) comprenant :

   un premier corps (122) qui partage un axe de pivotement avec un second corps (124), le premier corps étant rotatif par rapport au second corps pour permettre un premier mode de fonctionnement comprenant un mode de préhension et un deuxième mode de fonctionnement comprenant un mode de coupe,
   dans lequel le deuxième mode de fonctionnement est inopérant pendant le premier mode de fonctionnement, et dans lequel le premier mode de fonctionnement et le deuxième mode de fonctionnement peuvent être commandés sensiblement autour d'un axe ;
   un support non transitoire lisible par ordinateur (166) sur lequel sont stockées des instructions exécutables ; et
   un processeur (164) en communication avec le support non transitoire lisible par ordinateur et configuré pour exécuter les instructions afin d'amener le système à au moins :

   faire fonctionner l'effecteur terminal dans le premier mode de fonctionnement dans lequel le premier corps est rotatif par rapport au second corps dans une première plage de rotation afin d'exécuter une première fonction et d'empêcher une seconde fonction ;
   faire passer l'effecteur terminal du premier mode de fonctionnement au deuxième mode de fonctionnement ; et
   faire fonctionner l'effecteur terminal dans le deuxième mode de fonctionnement dans lequel le premier corps est rotatif par rapport au second corps dans une seconde plage de rotation différente de la première plage de rotation afin d'exécuter une seconde fonction et d'empêcher la première fonction.

2. Système médical selon la revendication 1, dans lequel le premier corps comporte une première surface de mise en prise ayant une première partie (128) décalée en rotation autour de l'axe de pivotement par rapport à une seconde partie (130), et dans lequel le second corps comporte une seconde surface de mise en prise ayant une première partie (128) décalée en rotation autour de l'axe de pivotement par rapport à une seconde partie (130).

3. Système médical selon la revendication 2, dans lequel le processeur est en outre configuré pour exécuter les instructions afin d'amener le système à au moins :

   faire fonctionner l'effecteur terminal dans le premier mode de fonctionnement pour permettre la coopération entre la première partie de la première surface de mise en prise et la première partie de la seconde surface de mise en prise et empêcher la coopération entre la seconde partie de la première surface de mise en prise et la seconde partie de la seconde surface de mise en prise, et
   faire passer l'effecteur terminal du premier mode de fonctionnement au deuxième mode de fonctionnement pour permettre la coopération entre au moins la seconde partie de la première surface de mise en prise et la seconde partie de la seconde surface de mise en prise.

4. Système médical selon la revendication 2, dans lequel la première partie du premier corps est décalée en rotation par rapport à la seconde partie du premier corps d'au moins 10 degrés.

5. Système médical selon la revendication 1, dans lequel le premier corps comporte une première partie (128) et une seconde partie (130), dans lequel la première partie comprend une partie de préhension et la seconde partie

23

comprend une partie de coupe.

6. Système médical selon la revendication 5, dans lequel, pendant le premier mode de fonctionnement, la partie de coupe n'est pas exposée.

7. Système médical selon la revendication 1, dans lequel le premier corps et le second corps comprennent tous deux une partie de préhension (128) et une partie de coupe (130), et dans lequel, pendant le premier mode de fonctionnement, les parties de coupe se chevauchent.

8. Système médical selon la revendication 7, dans lequel, pendant le deuxième mode de fonctionnement, un renfoncement est formé entre les parties de coupe.

9. Système médical selon la revendication 1, dans lequel, dans le premier mode de fonctionnement, l'effecteur terminal comprend une butée mécanique qui rend le deuxième mode de fonctionnement inopérant.

10. Système médical selon la revendication 9, dans lequel la butée mécanique comprend une butée dure formée sur le premier corps et le second corps de l'effecteur terminal.

11. Système médical selon la revendication 1, comprenant en outre un bras robotique accouplé à l'instrument chirurgical.

12. Système médical selon la revendication 1, dans lequel le processeur est en outre configuré pour exécuter les instructions afin d'amener le système à faire passer l'effecteur terminal du premier mode de fonctionnement ou du deuxième mode de fonctionnement à un troisième mode de fonctionnement dans lequel le premier corps est rotatif par rapport au second corps dans la première plage de rotation et la seconde plage de rotation afin d'exécuter la première fonction et la seconde fonction.

**FIG. 1**

EP 3 773 242 B1

**FIG. 2**

FIG. 3

**FIG. 4**

**FIG. 5**

**FIG. 6**

EP 3 773 242 B1

FIG. 7

FIG. 8

FIG. 9

**FIG. 10**

EP 3 773 242 B1

**FIG. 11**

**FIG. 12**

FIG. 13

EP 3 773 242 B1

**FIG. 14**

EP 3 773 242 B1

**FIG. 15**

**FIG. 16A**

FIG. 16B

**FIG. 17**

**FIG. 18A**     **FIG. 18B**     **FIG. 18C**

EP 3 773 242 B1

120

128

**FIG. 18D**

120

130

128

128

130

**FIG. 18E**

120

130

128

128

130

**FIG. 18F**

**FIG. 19**

160 ↘

120 ↘
| End Effector |

162 ↘
**Control System**
| Processor |

164 ↗

| Memory |

166 ↗

168 ↘
**Controller**
| Actuator |

170 ↗

| Mode Selector |

172 ↗

**FIG. 20A**

175 ↘

176 ↘
| IN A FIRST MODE, ROTATE A FIRST BODY RELATIVE TO A SECOND BODY IN A FIRST ROTATIONAL RANGE TO PERFORM A FIRST FUNCTION AND PRECLUDE A SECOND FUNCTION |

177 ↘
| TRANSITION FROM THE FIRST MODE TO A SECOND MODE |

178 ↘
| IN THE SECOND MODE, ROTATE THE FIRST BODY RELATIVE TO THE SECOND BODY IN A SECOND ROTATIONAL RANGE TO PERFORM THE SECOND FUNCTION |

**FIG. 20B**

**FIG. 21A**

EP 3 773 242 B1

FIG. 21B

150

152

156

154

**FIG. 22**

EP 3 773 242 B1

FIG. 23

50

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015313676 A1
- US 2013053877 A1
- US 5545170 A
- US 2007244515 A1
- US 523760